# EUROPEAN PATENT SPECIFICATION

(11) **EP 3 583 204 B1**
(45) Date of publication and mention of the grant of the patent: **08.07.2026**
(21) Application number: 18708345.6
(22) Date of filing: 16.02.2018
(51) Int. Cl.: C12N 5/079, C12N 5/0797

(54) **FEEDER-FREE METHODS FOR OBTAINING RETINAL PROGENITORS, RETINAL PIGMENTED EPITHELIAL CELLS AND NEURAL RETINAL CELLS**
FEEDER ZELLEN FREIE VERFAHREN ZUR HERSTELLUNG VON RETINA-VORLÄUFERN, PIGMENTIERTEN RETINA-EPITHELZELLEN UND NEURALEN RETINAZELLEN
PROCÉDÉ SANS CELLULES NOURRICIERES POUR OBTENIR DE PROGÉNITEURS RÉTINIENS, CELLULES ÉPITHÉLIALES PIGMENTAIRES RÉTINIENNES ET CELLULES NEURONALES RÉTINIENNES

(30) Priority: 17.02.2017 EP 17305181
(43) Date of publication of application: 25.12.2019
(73) Proprietor: Sorbonne Université, 75006 Paris (FR); Centre National de la Recherche Scientifique, 75016 Paris (FR); INSTITUT NATIONAL DE LA SANTE ET DE LA RECHERCHE MEDICALE (INSERM), 75013 Paris (FR)
(72) Inventor: REICHMAN, Sacha, 75003 Paris (FR); GOUREAU, Olivier, 75012 Paris (FR); SAHEL, José-Alain, 75013 Paris (FR); SLEMBROUK, Amélie, 78540 Venouillet (FR)
(74) Representative: Gevers & Orès
(86) International application number: PCT/EP2018/053923
(87) International publication number: WO 2018/149985

(56) References cited:
- WO-A1-2014/174492
- US-A1- 2014 134 732
- US-A1- 2016 175 362
- SACHA REICHMAN ET AL: "Production de rétines in vitro à partir de cellules pluripotentes humaines : Un nouvel outil thérapeutique", M/S MEDECINE SCIENCES., vol. 30, no. 10, 1 October 2014 (2014-10-01), FR, pages 845 - 848, XP055378611, ISSN: 0767-0974, DOI: 10.1051/medsci/20143010009
- BRITNEY O. PENNINGTON ET AL: "Defined Culture of Human Embryonic Stem Cells and Xeno-Free Derivation of Retinal Pigmented Epithelial Cells on a Novel, Synthetic Substrate : Synthetic Substrate for Stem Cell and RPE Culture", STEM CELLS TRANSLATIONAL MEDICINE : SCTM, vol. 4, no. 2, 15 January 2015 (2015-01-15), Durham, pages 165 - 177, XP055378623, ISSN: 2157-6564, DOI: 10.5966/sctm.2014-0179

## Description

The impaired or complete loss of function of photoreceptor cells or supporting retinal pigmented epithelium (RPE) is the main cause of irreversible blindness in retinal diseases, such as inherited retinopathies and age-related macular degeneration (AMD). The death of retinal ganglion cells (RGCs) in glaucoma also results in irreversible loss of vision. Cell replacement strategies using cell derivatives of pluripotent stem cells are very promising approaches to rescue the degenerated retina. Stepwise differentiation protocols were designed to mimic retinal differentiation and to successfully generate RPE cells, RGCs and photoreceptors from human embryonic stem (hES) cells and induced pluripotent stem (hiPS) cells [1-14]. Recent processes demonstrated that hESs or hiPSs could efficiently form 3D retinal structures, starting from embryoid bodies [15-18] or simply from confluent hiPS cultures [19, 20]. *In vitro* methods for obtaining substantially pure cultures of certain human neuroepithelial lineage cells, including retinal progenitor cells, RPE cells and neural retinal cells, have already have been described, such as in WO 2014/174492.

The majority of these approaches relies on animal-derived components in the medium and animal-derived substrate or the presence, in the *in vitro* culture, of feeder cells. Basically, feeder cells consist in a layer of cells unable to divide (thereby also designated as "growth-arrested cells"), which provides extracellular secretions and in particular growth factors, and are typically used to promote cell proliferation and differentiation.

However, the clinical application of cell derivatives of pluripotent stem cells is hindered by several of the culture parameters that make obtaining such cell derivatives actually possible. The greatest of these obstacles is the requirement of mouse/human-derived feeder cells. Indeed, the feeder cells deliver an unknown collection of factors, which, despite being mandatory in the growth and differentiation of pluripotent stem cells, pose a real risk of transferring animal/human viruses to the cells, and ultimately the patient.

Therefore, the establishment of a feeder-free (FF) method (i.e. not involving the use of feeder cells), advantageously also xeno-free (XF, i.e. wherein all components of the cell culture medium are derived from the same organism), following current Good Manufacturing Practice (GMP) guidelines for the generation of transplantable retinal cell types derived from hESCs or hiPSCs is needed for future clinical applications.

The use of a feeder-free (FF) system with chemically defined media has been described for the culture hES cells and hiPS cells [21]. However XF/FF culture conditions are poorly documented for differentiation towards retinal neurons [26, 27].

As disclosed in the experimental part below, the inventors have now subjected iPS cells to a new retinal differentiation protocol, based on a feeder-free culture system and advantageously in the absence of xenogenic products. This protocol avoids the formation of embryoid bodies or cell clumps, and can be performed in absence of matrigel or serum. The inventors demonstrated that adherent hiPS cells cultured in a stem-cell-specific pro-neural medium can generate within three to five weeks neuroepithelial-like structures with an eye field identity, which, when switched to 3D cultures, can differentiate into the major retinal cell types. Under these conditions, hiPS cells self-assembled into neural retina-like tissues, with rapid expression of retinal markers in a developmentally appropriate time window; they gave rise to different retinal cell types such as RGCs and photoreceptors.

A first object of the present invention is hence an *in vitro* method for obtaining human retinal progenitors, comprising the steps of:
(i) placing an adherent culture of human pluripotent stem cells into a stem-cell-specific pro-neural medium, said culture being devoid of feeder cells; and
(ii) maintaining this culture in said stem-cell-specific pro-neural medium until the appearance of pigmented cells and/or of neuroepithelial-like structures.

In the present text, the "retinal progenitors", also called "retinal progenitor cells", encompass cells which are competent for generating all cell types of the neural retina, including precursors of photoreceptors, as well as cells which can differentiate into RPE.

"Human pluripotent stem cells" include human embryonic stem (hES) cells and human induced pluripotent stem (hiPS) cells. Such cells may easily been obtained using methods known in the art, for instance the methods which do not imply the destruction of embryos detailed by Chung *et al*. [45]. The above method is advantageously performed with human induced pluripotent stem cells.

"Feeder cells" herein refer to growth-arrested cells. "Feeder cells" encompass fibroblasts, particularly human foreskin fibroblasts, adult dermal fibroblasts and primary mouse embryonic fibroblasts are generally used in the field for the culture of stem cells.

As previously indicated, the method of the invention is feeder-free, and thus does not require the use of feeder cells.

In a preferred embodiment of the above method, the culture of human pluripotent stem cells and the stem-cell-specific pro-neural medium are devoid of feeder cells. In a yet preferred embodiment, the culture of human pluripotent stem cells and the stem-cell-specific pro-neural medium are devoid of fibroblasts, advantageously devoid of human foreskin fibroblasts, adult dermal fibroblasts and primary mouse embryonic fibroblasts. Advantageously, the culture of human pluripotent stem cells and the stem-cell-specific pro-neural medium are devoid of any cell not derived from human pluripotent stem cells.

Advantageously, the above method can be performed in the absence of xenogenic products. According to a preferred embodiment of the above method, all components of the stem-cell-specific pro-neural medium are derived from the same organism, advantageously all components of the stem-cell-specific pro-neural medium are derived from human origin. According to a yet preferred embodiment of the above method, all proteins of the stem-cell-specific pro-neural medium are derived from human origin. In the context of the invention, it should be understood that this definition encompasses both components isolated from human samples, more particularly proteins, and recombinant human components, such as recombinant proteins. When recombinant human proteins are used, they may be produced in organisms other than human cells, provided the nucleic acid sequence used is of human origin, or derived from a sequence of human origin.

A "pro-neural medium" generally designates a culture medium which favors the maintenance and/or growth of neuronal cells. It is typically composed of a nutrient medium said nutrient medium being supplemented with a pro-neural supplement which comprises at least part of the following elements: carbon sources, vitamins, inorganic salts, amino acids and a protein digest.

The inventors have however demonstrated that the differentiation of human pluripotent stem cells into human retinal progenitors, when using a feeder-free *in vitro* system, requires the use of a specific pro-neural medium, adapted to stem cells.

In the context of the invention, the "stem-cell-specific pro-neural medium" comprises a stem-cell-specific nutrient cell medium, and a pro-neural supplement which comprises at least part of the following elements: carbon sources, vitamins, inorganic salts, amino acids and a protein digest. The person skilled in the art may easily define the relative proportions of the stem-cell-specific nutrient cell medium and of the pro-neural supplement. Preferably, the volume of the pro-neural supplement represents 1% to 2% of the final volume of the stem-cell-specific pro-neural medium.

An appropriate stem-cell-specific nutrient cell medium, named E8 medium, commercialized as Essential 8^{™} medium by the company Thermo Fischer Scientific, has been described by Chen et al. [21]. E8 medium consists in insulin, selenium, transferrin, L-ascorbic acid, FGF2, and TGFβ (or NODAL) in DMEM/F12 with pH adjusted with NaHCO₃. More precisely, this medium has been defined in Chen et al. as follows: E8 medium contains DMEM/F12, L-ascorbic acid-2-phosphate magnesium (64 mg/l), sodium selenium (14 µg/l), FGF2 (100 µg/l), insulin (19.4 mg/l), NaHCO₃ (543 mg/l) and transferrin (10.7 mg/l), TGFβ1(2 µg/l) or NODAL (100 µg/l), wherein osmolarity of the medium is adjusted to 340 mOsm at pH7.4.

Stem-cell-specific nutrient cell media derived from the E8/Essential 8^{™} medium, such as E7 medium, commercialized as Essential 7^{™} (Thermo Fischer Scientific) or E6 medium, commercialized as Essential 6^{™} (Thermo Fischer Scientific), may also be used. E7 medium has a similar composition than that of E8 medium, but does not contain any TGFβ (i.e., is devoid of TGFβ). E7 medium consists in insulin, selenium, transferrin, L-ascorbic acid, FGF2, in DMEM/F12 with pH adjusted with NaHCO₃. E6 medium has a similar composition than that of E8 medium, but is devoid of TGFβ and devoid of FGF2. E6 medium consists in insulin, selenium, transferrin, L-ascorbic acid, in DMEM/F12 with pH adjusted with NaHCO₃.

Preferably, the stem-cell-specific nutrient cell medium consists in insulin, selenium, transferrin, L-ascorbic acid, in DMEM/F12 with pH adjusted with NaHCO₃, optionally further comprising TGFβ and/or FGF2.

Commercial stem-cell-specific nutrient cell media such as TeSR^{™}-ES(STEMCELL Technologies), TeSR^{™}-E7 (STEMCELL Technologies), TeSR^{™}-E6 (STEMCELL Technologies), NutriStem (STEMGENT) and iPS-Brew, in particular the medium commercialized as StemMACS iPS-Brew XF Basal medium (Miltenyi) may also be used.

An appropriate pro-neural supplement for obtaining a stem-cell-specific pro-neural medium may be chosen among well-known supplements such as N2, B27, G5 and BIT9500 supplements, as well as any supplement derived from these. The components present in these supplements are summarized in Table 1 below.

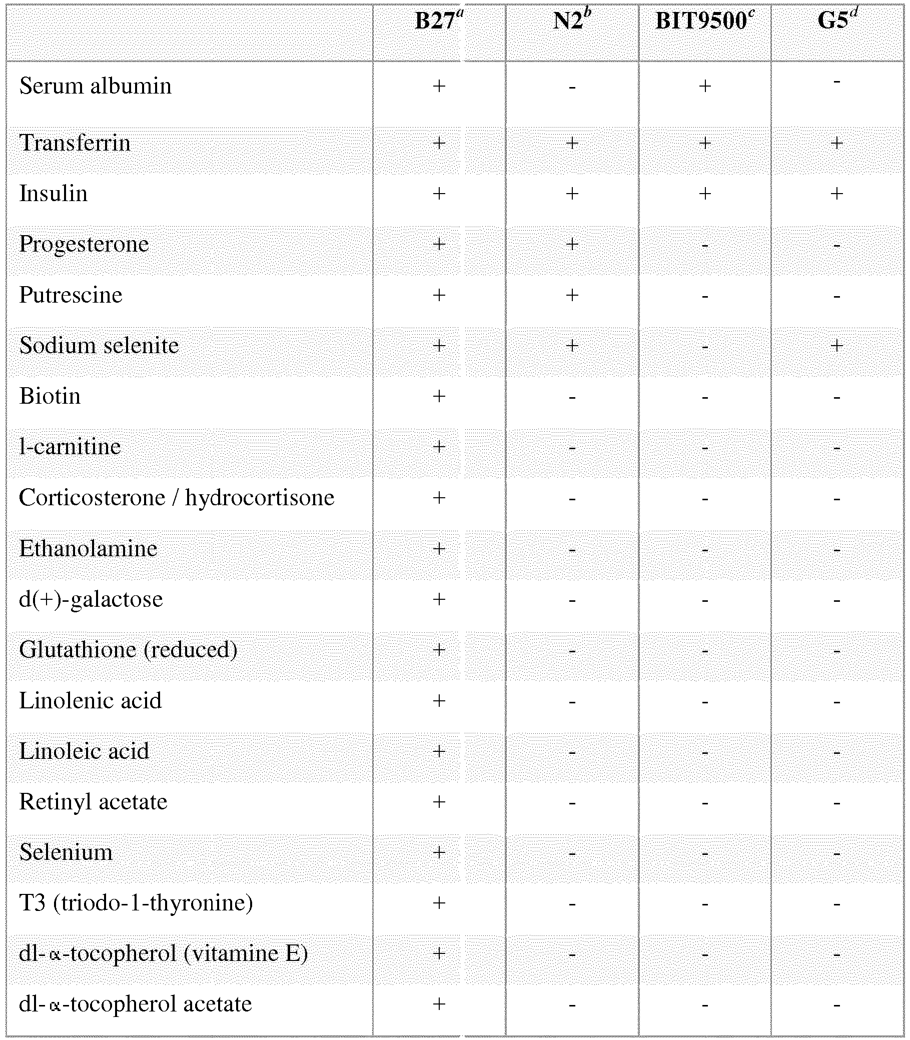

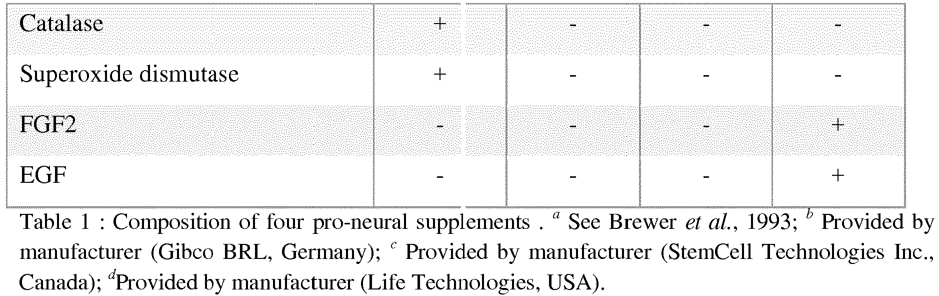

However, when the above method is to be performed "xeno-free", that is to say in the absence of xenogenic products, the pro-neural supplement should be formulated only with components derived from the same organism, also herein called "xeno-free pro-neural supplement". Thus, in an embodiment of the above method, all components of the pro-neural supplement are derived from the same organism, preferably all components of the pro-neural supplement are derived from human origin. Preferably, in such embodiment, the pro-neural supplement is chosen among N2 supplement and B27 supplement, wherein all components of the pro-neural supplement for are derived from the same organism, yet preferably derived from human origin.

Appropriate xeno-free pro-neural supplement are for instance the B-27^{®} Supplement XenoFree CTS^{™} (Thermo Fisher Scientific), which is a complete B-27^{®} serum-free supplement formulated with only recombinant or humanized components, and the N2^{®} Supplement XenoFree CTS^{™} (Thermo Fisher Scientific) which is a complete N2^{®} serum-free supplement formulated with only recombinant or humanized components.

In an embodiment of the above method, the stem-cell specific pro-neural medium is devoid of serum albumin. In such an embodiment, the use of a pro-neural supplement such as N2 is therefore preferred for the preparation of the stem-cell specific pro-neural medium.

Preferably, the stem-cell-specific pro-neural medium used in the method of the invention comprises a stem-cell-specific nutrient cell medium, such as one of the media listed above, and N2 supplement, yet preferably wherein all components of the pro-neural supplement are derived from the same organism, advantageously derived from human origin.

In an embodiment, the stem-cell-specific pro-neural medium used in the method of the invention comprises or consists of progesterone, putrescine, sodium selenite, insulin, selenium, transferrin, L-ascorbic acid, in DMEM/F12 with pH adjusted with NaHCO₃, optionally further comprising TGFβ and/or FGF2, yet preferably wherein all components of the stem-cell-specific pro-neural medium are derived from the same organism, advantageously derived from human origin.

It is known in the art that FGF2 contributes to maintaining pluripotent stem cells in a pluripotent state, thereby preventing differentiation. Thus, preferably, the stem-cell-specific pro-neural medium used is devoid of FGF2. Advantageously, the stem-cell-specific pro-neural medium used in the method of the invention comprises a nutrient medium having the composition of E6, and the N2 supplement. In such embodiment, the stem-cell-specific pro-neural medium used in the method of the invention comprises or consists of progesterone, putrescine, sodium selenite, insulin, selenium, transferrin, L-ascorbic acid, in DMEM/F12 with pH adjusted with NaHCO₃, yet preferably wherein all components of the stem-cell-specific pro-neural medium are derived from the same organism, advantageously derived from human origin.

Advantageously, the above method can be performed without using differentiation factors. According to a preferred embodiment of the above method, the stem-cell-specific pro-neural medium used is devoid of at least one of the following differentiation factors: noggin, Dkk-1 and IGF-1. In particular, the pro-neural medium can be devoid of these three factors.

The human pluripotent stem cells used in step (i) can be cultured in any kind of adherent culture system. Non-limitative examples of surfaces which can be used for this culture are: glass, plastic (possibly treated), vitronectin, alanin, collagen, laminin, fibronectin, Geltrex^{™}, Cellstart^{™}, Matrigel^{™}, poly-L-lysin, nutrient cells, or any synthetic surface commercially available such as Corning Synthemax^{™}. Preferably, the surface used in the *in vitro* system comprises or consists of at least a protein of human origin, such as for instance vitronectin, alanin, collagen, laminin, fibronectin, or poly-L-lysin, In the context of the invention, when the culture is to be xeno-free, the surface used in the *in vitro* system preferably comprises or consists of at least a protein of human origin, such as for instance human vitronectin, human collagen, human laminin, human fibronectin. By "molecules of human origin" it is herein referred to molecules having the peptidic sequence of the corresponding molecule found in human. Such "molecules of human origin" can easily be obtained by isolation of the molecule from human samples, or by the production of recombinant molecules. In the context of the invention, functional truncated proteins or functional protein variants may also be used. By "functional truncated proteins" and "functional protein variants", it is herein referred to a truncated protein or protein variant which retains the ability of the whole protein to facilitate the adhesion of pluripotent stem cells, which can easily be verified by the person skilled in the art.

Vitronectin in particular has been shown to facilitate the culture of pluripotent cells. Thus, advantageously, in the context of the invention, the human pluripotent stem cells used in step (i) are cultured in the presence of vitronectin, preferably human vitronectin, yet preferably truncated human vitronectin. Advantageously, the human pluripotent stem cells used in step (i) are cultured in the presence of a recombinant truncated human vitronectin corresponding to the amino-acid fragment 62-478 of human vitronectin. The product VTN-N, commercialized by Thermo Fischer Scientific or STEMCELL technologies may for instance be used for that purpose.

As described in the experimental part, and although this is not compulsory, the method according to the present invention can be performed so that step (i) is preceded by a step of adherent culture of said pluripotent stem cells in a culture medium for maintenance of pluripotent stem cells, during 1 to 4 days, preferably during 2 days. Preferably, the above step of adherent culture of said pluripotent stem cells in a culture medium for maintenance of pluripotent stem cells is realized using a culture medium for maintenance of pluripotent stem cells modified so that it is devoid of basic fibroblast growth factor (bFGF/FGF2), during 1 to 4 days, preferably during 2 days. Non-limitative examples of appropriate media devoid of basic fibroblast growth factor (bFGF/FGF2), for this additional step are the Essential 6^{™} medium , the TeSR^{™} -E6 medium and the medium commercialized as StemMACS iPS-Brew XF Basal medium by Miltenyi Biotech. Alternatively, the above step of adherent culture of said pluripotent stem cells in a culture medium for maintenance of pluripotent stem cells is realized using a culture medium for maintenance of pluripotent stem cells comprising basic fibroblast growth factor (bFGF/FGF2), 1 to 4 days, preferably during 2 days. Non-limitative examples of appropriate media comprising basic fibroblast growth factor (bFGF/FGF2), for this additional step are the Essential 8^{™} medium, the TeSR^{™} -E8 medium, and the StemMACS iPS-Brew XF complete medium (StemMACS iPS-Brew XF Basal medium supplemented StemMACS iPS-Brew XF Supplement).

According to the invention, step (ii) is performed until the appearance of pigmented cells and/or of neuroepithelial-like structures.

In what precedes, "neuroepithelial-like structures", also named "neural retina-like structures" in the experimental part below, designate phase-bright structures which start to appear after a few days of culture in a pro-neural medium. These structures are essentially made of cells which do not significantly express pluripotency-related genes such as *OCT4*, and which express transcription factors associated with eye-field specification such as *LHX2*, *RAX*, *PAX6* and *SIX3*. As disclosed in the experimental part, when performing the above method, pigmented cells first appear, and the neuroepithelial-like structures most often appear in the vicinity of a patch of pigmented cells.

Of course, when performing the methods according to the present invention, the skilled artisan can detect the expression of various markers (to check either their expression or the fact that they are not expressed anymore, and/or to quantitatively measure their expression level). Any technique known in the art can be used to this aim, such as, for example, quantitative RT-PCR and immunoassays. Examples of markers for pluripotency are OCT4, SOX2 and NANOG; examples of markers for the eye field are RAX, PAX6, OTX2, LHX2 and SIX3, the two first ones being preferred.

The inventors have observed that the confluence of the cells in the adherent culture of the invention may play a role in obtaining viable pigmented cells and/or of neuroepithelial-like structures. Indeed, if and when the adherent culture reaches 80% confluence, the pigmented cells and/or neuroepithelial-like cells obtained are of a lesser quality and their viability is affected. In cell culture biology, confluence is the term commonly used as an estimate of the number of adherent cells in a culture dish or a flask, referring to the proportion of the surface which is covered by cells. The skilled artisan is familiar with the notion of confluence for adherent cells, and will be able to evaluate this confluence, which can be appreciated locally, *i*.*e*. only in one area of the recipient, especially if the confluence is non homogeneous on the whole culture surface. In the case of colony-type monolayers, a "80% confluence" can be defined, if needed, as the situation when some colonies punctually come into contact with other colonies, while some free space (representing between 10 and 30% of the surface) remains between these colonies.

In a preferred embodiment, step (ii) is performed until the adherent culture reaches 60-80%, advantageously 70-80% confluence. Preferably, in the method of the invention, the confluence of the cells is inferior to 80%.

In a particular embodiment of the above method, step (ii) is performed during at least 16 days and preferably between 19 to 33 days, so that a sufficient amount of neuroepithelial-like structures appear. Of course, the method of culture may evolve so that step (ii) can be shortened. As already mentioned above, the neuroepithelial-like structures are essentially made of cells which do not significantly express pluripotency-related genes such as *OCT4*, and which express transcription factors associated with eye-field specification. Hence, depending on the culture system, the skilled artisan can chose to define the end of step (ii) as the time when at least some cells stop expressing *OCT4* and/or start expressing *RAX* and *PAX6*. As already mentioned, this characterization can be performed by any known technique, such as qRT-PCR or immunostaining.

According to another aspect, the present invention pertains to a method for obtaining RPE cells, wherein said method comprises the steps of:
(i) placing an adherent culture of human pluripotent stem cells into a stem-cell-specific pro-neural medium, said culture being devoid of feeder cells;
(ii) maintaining this culture in said stem-cell-specific pro-neural medium until the appearance of pigmented cells;
(iii_{RPE}) collecting, from the culture obtained in step (ii), at least one pigmented cell; and
(iv_{RPE}) culturing the pigmented cell(s) obtained in step (iii_{RPE}).

When performing this method, the skilled artisan can check that the cells collected in step (iii_{RPE}) express the microphthalmia-associated transcription factor (MITF) and/or ZO-1. As already mentioned, any technique known in the art (such as qRT-PCR and immunostaining) can be used to this aim.

According to a preferred embodiment of the above method for obtaining RPE cells, the culture in step (iv_{RPE}) is carried out in an adherent culture system. Any adherent culture system can be used, as already mentioned above.

When performing the method of the invention for obtaining RPE cells, the cells are amplified in step (iv_{RPE}) during at least 5 days. Advantageously, the culture of step (iv_{RPE}) can be maintained and amplified during several weeks, to obtain great amounts of RPE cells: for example, when about 10 patches of pigmented cells are collected in step (iii_{RPE}) and plated together in a new dish of 3 cm², a substantially pure (99%) confluent adherent culture of RPE cells is obtained after 2 to 4 weeks, or after 10 to 14 days if FGF2 is added to the culture medium (10 ng/ml every 2 to 3 days).

The step (iv_{RPE}) of culturing the pigmented cell(s) obtained in step (iii_{RPE}) may be performed using any type of pro-neural medium, and is not limited to the stem-cell-specific pro-neural medium described above, although stem-cell-specific pro-neural may also be used. Non-limitative examples of appropriate pro-neural media to perform this step are any medium composed of a nutrient medium, such as Dulbeco's Modified Eagle Medium: Nutrient Mixture F-12 (DMEM/F12) or Neurobasal^{®} Medium (Gibco^{®}), said nutrient medium being supplemented with a pro-neural supplement as defined above, such as N2, B27, G5 and BIT9500 supplements, as well as any supplement derived from these.

Preferably the step (iv_{RPE}) of culturing the pigmented cell(s) obtained in step (iii_{RPE}) is performed using Dulbeco's Modified Eagle Medium: Nutrient Mixture F-12 (DMEM/F12) supplemented with N2 supplement, advantageously further supplemented with 1% MEM non-essential amino-acids. Preferably, in step (iv_{RPE}) of culturing the pigmented cell(s) obtained in step (iii_{RPE}), all components of the pro-neural medium are derived from the same organism, advantageously derived from human origin.

Another aspect of the present invention is a method for obtaining neural retinal cells, wherein said method comprises the steps of:
(i) placing an adherent culture of human pluripotent stem cells into a stem-cell-specific pro-neural medium, said culture being devoid of feeder cells;
(ii) maintaining this culture in said stem-cell- specific pro-neural medium until the appearance of neuroepithelial-like structures;
(iii_{NR}) collecting, from the culture obtained in step (ii), cells from at least one neuroepithelial-like structure; and
(iv_{NR}) culturing the cells obtained in step (iii_{NR}).

The "neural retinal cells" herein include RGC, bipolar cells, horizontal cells, amacrine cells, photoreceptor cells (rod and cones), Müller glial cells as well as precursors of any of these cell types.

The step (iv_{NR}) of culturing the cell(s) obtained in step (iii_{NR}) may be performed using any type of pro-neural medium, and is not limited to the stem-cell-specific pro-neural medium described above, although stem-cell-specific pro-neural may also be used. Non-limitative examples of appropriate pro-neural media to perform this step are any medium composed of a nutrient medium, such as Dulbeco's Modified Eagle Medium: Nutrient Mixture F-12 (DMEM/F12) or Neurobasal^{®} Medium (Gibco^{®}), said nutrient medium being supplemented with a supplement appropriate for obtaining a pro-neural medium such as N2, B27, G5 and BIT9500 supplements, as well as any supplement derived from these.

Preferably the step (iv_{NR}) of culturing the cell(s) obtained in step (iii_{NR}) is performed using Dulbeco's Modified Eagle Medium: Nutrient Mixture F-12 (DMEM/F12) supplemented with B27 supplement, advantageously further supplemented with 1% MEM non-essential amino-acids. Preferably, in step (iv_{NR}) of culturing the cell(s) obtained in step (iii_{NR}), all components of the pro-neural medium are derived from the same organism, advantageously derived from human origin.

Importantly, the various neural retinal cells do not appear at the same time during step (iv_{NR}), during which the cultured cells differentiate. Hence, depending on the duration of step (iv_{NR}), different cell types will form. The order of appearance is as follows: ganglion cells appear first, followed by amacrine cells and horizontal cells, and photoreceptors appear later. Depending on the cell-type which is needed, the skilled artisan will hence perform the culturing step (iv_{NR}) for as long as necessary, and for up to around 9 month (about 290 days).

As exemplified in the experimental part below, the method according to this aspect of the invention can be performed by collecting, in step (iii_{NR}), at least one neuroepithelial-like structure. This can be done, for example, by mechanically separating this structure from the layer of adherent cells. This structure can then be placed, either alone or together with other neuroepithelial-like structures, in another culture recipient, such as a well of a multiwell plate, a Petri dish, a flask, *etc*.

When performing this method, the skilled artisan can advantageously check that the cells collected in step (iii_{NR}) co-express PAX6 and RAX, characteristic of eye field cells. Alternatively or additionally, the expression of the cell proliferation marker Ki67 by the cells collected in step (iii_{NR}) can be measured.

According to a particularly advantageous aspect, the present invention pertains to a method for obtaining photoreceptor precursors, comprising the above steps (i) to (iv_{NR}), wherein step (iv_{NR}) is performed during at least 21 days, preferably at least 28 days. Of course, depending on the future development of the culture conditions, this step may be further shortened.

At any time during step (iv_{NR}), the skilled artisan can check the differentiation into the photoreceptor lineage by measuring the expression of *NRL* and/or *CRX* in the cultured cells, for example by qRT-PCR. Alternatively or in addition, photoreceptor precursors can be identified with a RECOVERIN immunostaining, as disclosed in the experimental part below. The inventors have also demonstrated that CD73, which can be used as a cell surface marker for cell sorting of photoreceptor precursors, is co-expressed with RECOVERIN. This can advantageously be used by adding a further step of cell sorting of photoreceptor precursors following step (iv_{NR}), for example by using an anti-CD73 antibody. The resulting cell population, enriched in photoreceptor precursors, can be used, for example, for cell transplantation or screening approaches.

Optionally, a Notch inhibitor such as DAPT can be added to the culture medium during at least 1 day, preferably during 5 days or more in step (iv_{NR}). DAPT is a γ-secretase inhibitor and indirectly an inhibitor of Notch, and the inventors have shown that its addition during a few days in step (iv_{NR}) favors the generation of photoreceptor precursors.

According to a preferred embodiment of the method for obtaining neural retinal cells disclosed above, the culture in step (iv_{NR}) is carried out in a non-adherent culture system. For example, neuroepithelial-like structures collected in step (iii_{NR}) are cultured as floating structures. According to a specific embodiment, each neuroepithelial-like structure collected in step (iii_{NR}) is cultured in an individual recipient/well as a floating structure.

Non limitative examples of non-adherent systems include magnetically rotated spinner flasks or shaken flasks or dishes in which the cells are kept actively suspended in the medium, as well as stationary culture vessels or T-flasks and bottles in which, although the cells are not kept agitated, they are unable to attach firmly to the substrate.

As described in the experimental part, the cells or neuroepithelial-like structures can advantageously be kept actively suspended in the medium by performing step (iv_{NR}) under shaking conditions. Any shaker can be used for this purpose, such as, for example, a rotator which agitates the cultures in three dimensions.

According to another preferred embodiment of the method of the invention for obtaining neural retinal cells, the culture medium used in step (iv_{NR}) is supplemented with FGF2 during at least 5 days. This culture medium is preferably a pro-neuronal medium as defined above.

One advantage of the present invention is that, from a first adherent culture, two different cultures can be performed in parallel in order to obtain both RPE cells (first culture, preferably adherent) and precursors of the neural retina (second culture, preferably non-adherent). Accordingly, the present invention pertains to a method for obtaining both RPE cells and precursors of the neural retina, comprising steps (i) and (ii) as defined above, followed by steps (iii_{RPE}) and (iv_{RPE}) defined above, performed in parallel with steps (iii_{NR}) and (iv_{NR}) also disclosed above.

Most importantly, the present invention provides reliable methods to easily and rapidly obtain large amounts of retinal cells of any of the major types (RPE, RGCs, amacrine cells, horizontal cells, Müller glial cells and photoreceptors), with a high degree of purity.

It is envisioned that these methods, and the substantially pure cell cultures obtained through them, are useful in the following areas:
- Transplantation / cell therapy: non-limiting examples include the use of RPE cells and/or of retinal progenitor cells or cells differentiated therefrom, in lost cells replacement therapy to help restore previously lost vision. The methods and cultures could also be used for developing tissues for use in whole tissue replacement therapy.
- Drug screening for identifying agents able to protect or enhance the function of all cells, including RGCs, rods, cones and RPE cells. By "agent" is herein meant any kind of molecule or composition, but also non-chemical agents such as any electromagnetic or corpuscular radiation (UV, visible light, ionizing radiation, *etc*.).
- Producing human retinal disease models from pluripotent cells, especially from hiPS cells, which can also be used to study pathophysiology and for drug screening or customized therapy using stem cells or derivatives thereof.
- As a unique model of human neural development, which would be a useful resource to study a variety of processes, including without limitation retinal development, tissue formation, and synapse formation.

The invention is further illustrated by the following figures and example.

### LEGENDS OF THE FIGURES

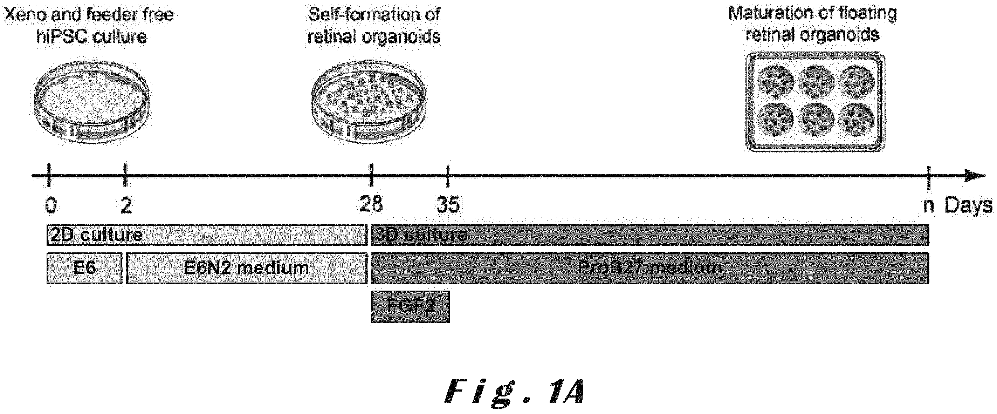

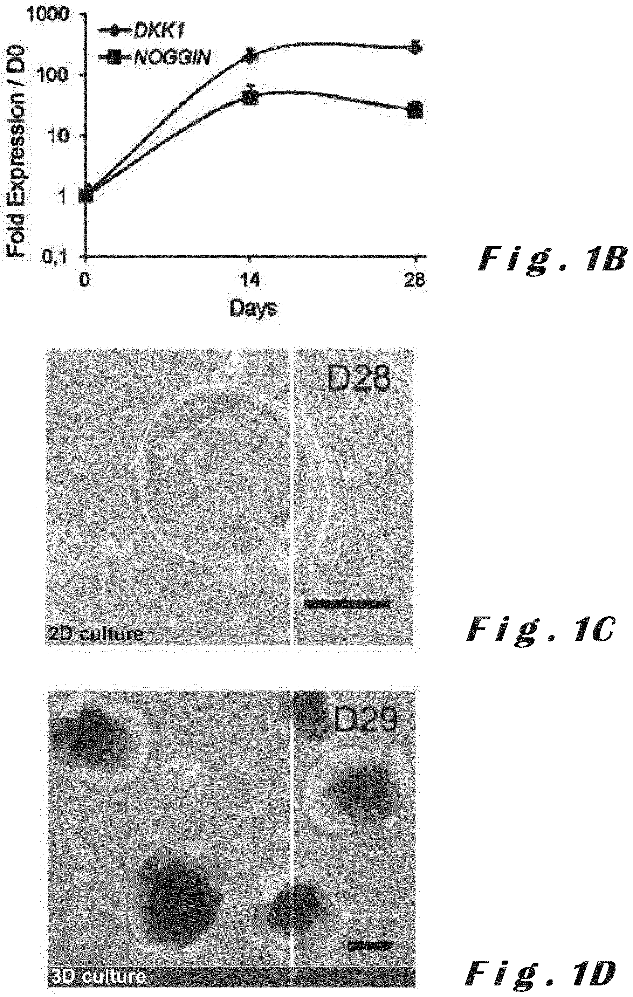

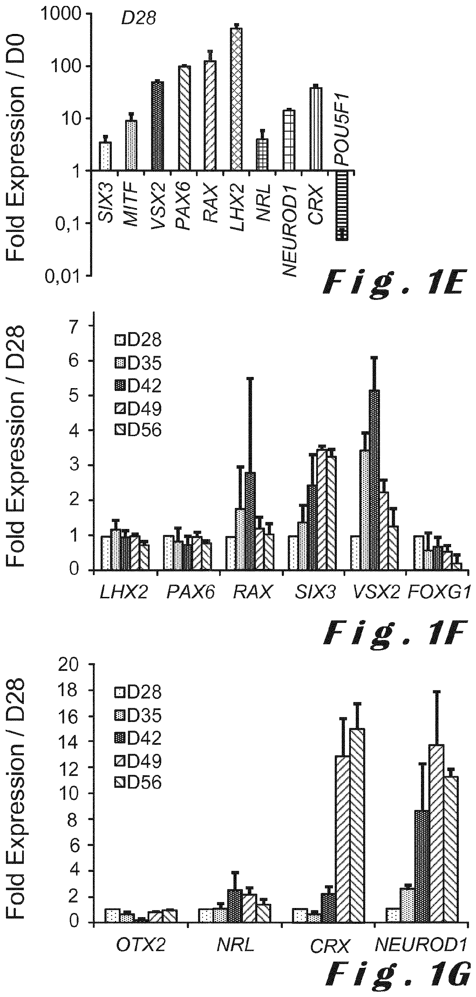

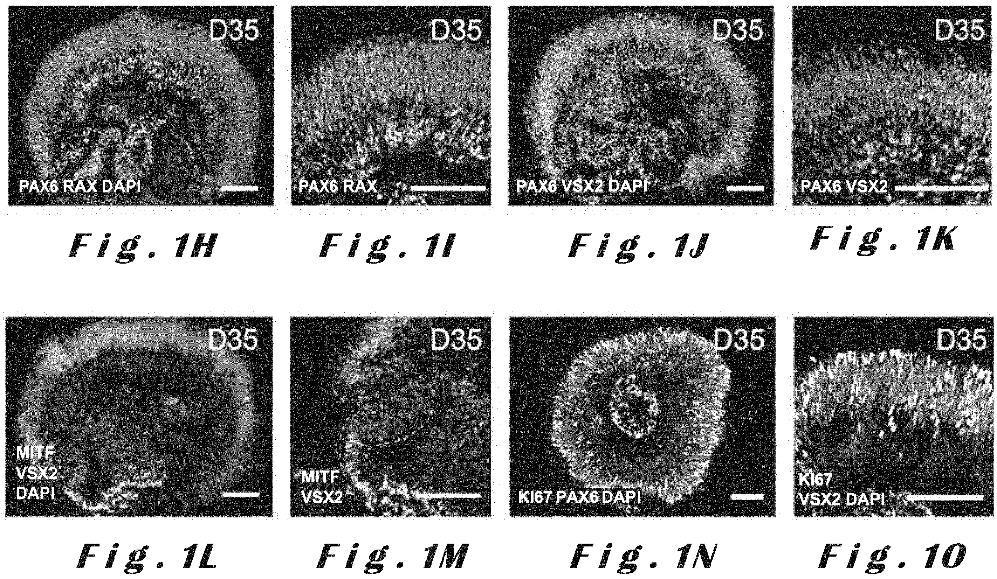

**Figure 1****.** Generation of retinal organoids from adherent hiPSCs in Xeno Free and Feeder Free conditions.
   (**A**) Schematic diagram illustrating the protocol for retinal organoid production from hiPSCs. (**B**) RT-qPCR analysis of DKK1 and NOGGIN during differentiation at D0, D14 and D28. Data are normalized to hiPSC-2 at D0. (C) Self-forming neuroretinal-like structures derived from adherent hiPSCs at D28. Scale bar = 100 µm. (**D**) Morphology of representative floating retinal organoids isolated at D29. Scale bars= 100 µm. (**E**) RT-qPCR analysis of eye-field transcription factors, NRL, CRX, NEUROD1 and pluripotency marker POU5F1 in retinal organoids at D28. Data are normalized to hiPSC-2 at D0. (**F-G**) RT-qPCR time course analysis of eye-field and retinal specific transcription factors and forebrain marker FOXG1 in retinal organoids between D28 to D56. Data are normalized to retinal organoids at D28. (**H-O**) Immunostaining of retinal organoid sections at D35 for PAX6, RAX, VSX2, MITF and Ki67. Picture in panel N correspond to a transverse section of retinal organoids. Scale bars = 100 µm.

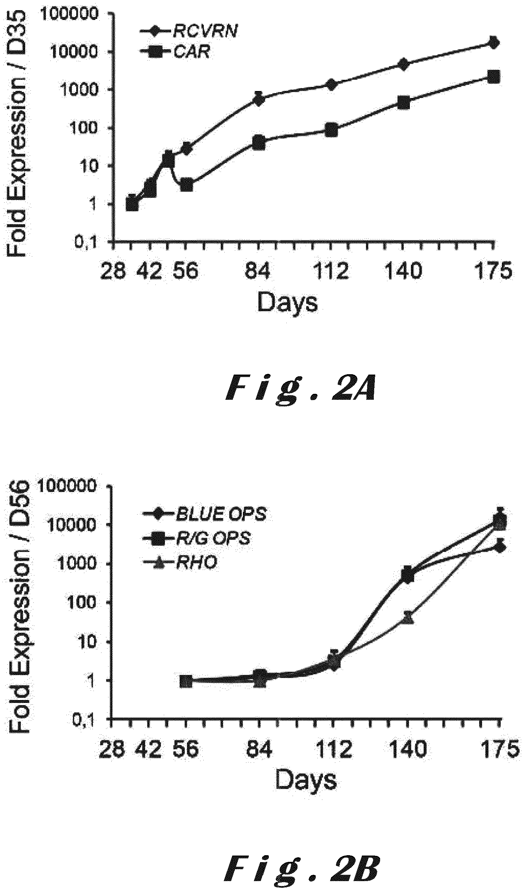

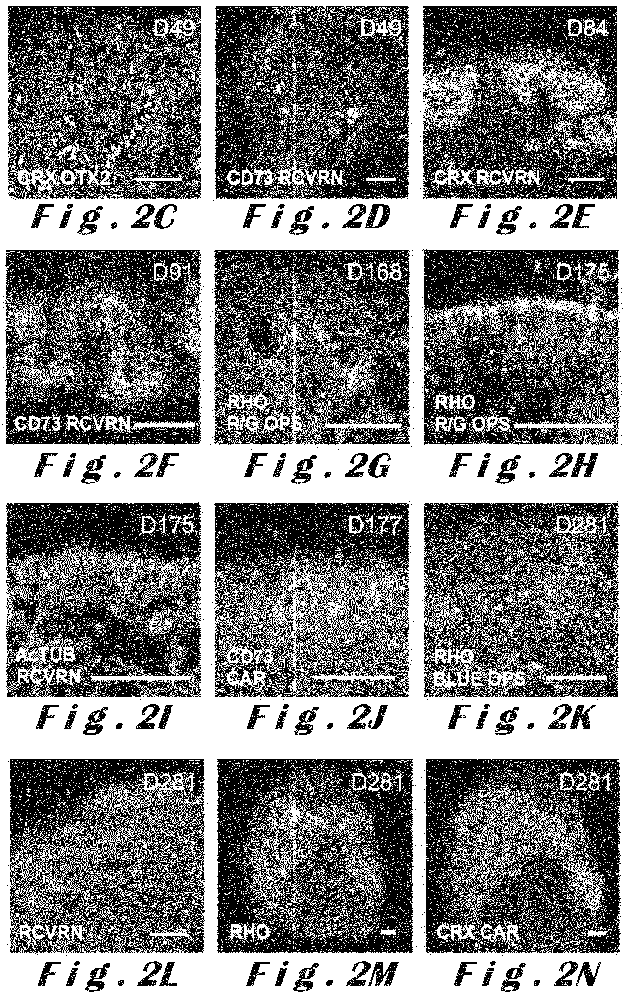

**Figure 2****.** Photoreceptor differentiation in retinal organoids during floating cultures.
   (**A-B**) RT-qPCR analysis of photoreceptor markers during differentiation between D35 to D175. Data are normalized to retinal organoids at D35 (A) or D56 (B). (**C-N**) Immunostaining of retinal organoid sections for CRX (C, E, N), OTX2 (C), CD73 (D, F, J), RECOVERIN (D-F, I, L), RHODOPSIN (G, H, K, M), R/G OPSIN (H), BLUE OPSIN (K), Acetylated TUBULIN (I) and CONE ARRESTIN (J, N) at the indicated time of differentiation. Nuclei were counterstained with DAPI (blue). Scale bars = 50 µm (C, D, G-I) or 100 µm (E, F, J-N).

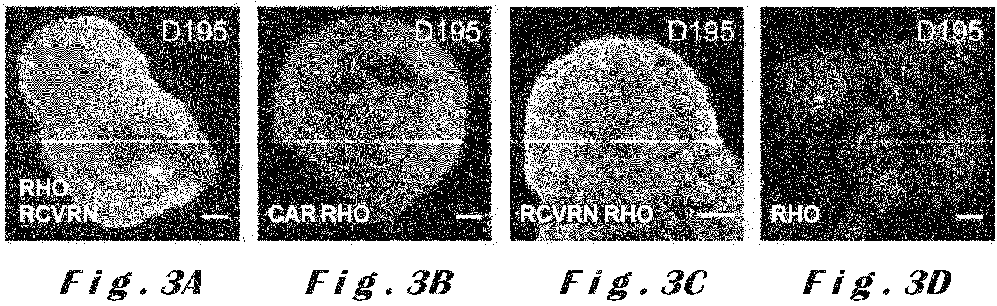

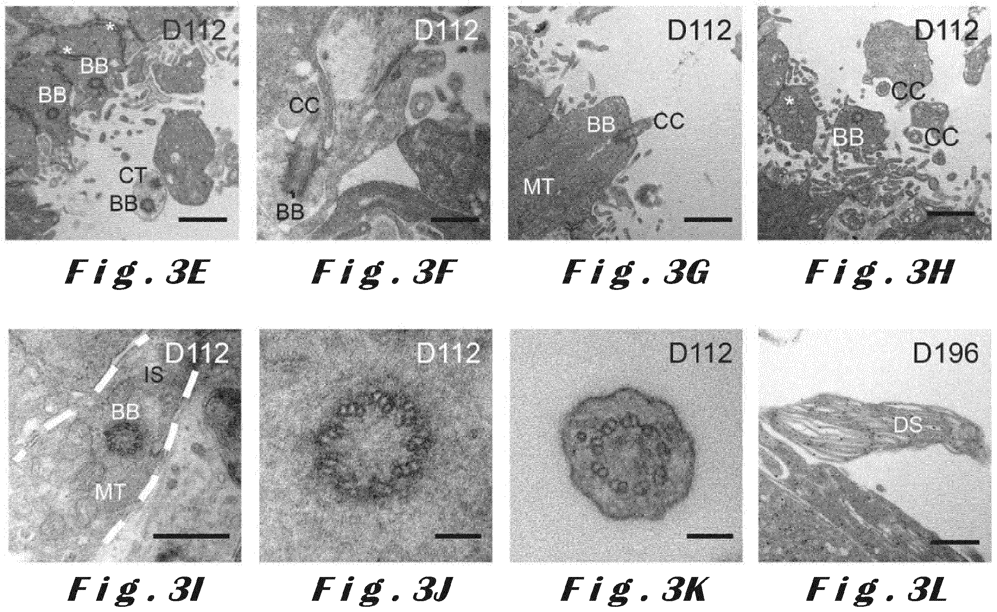

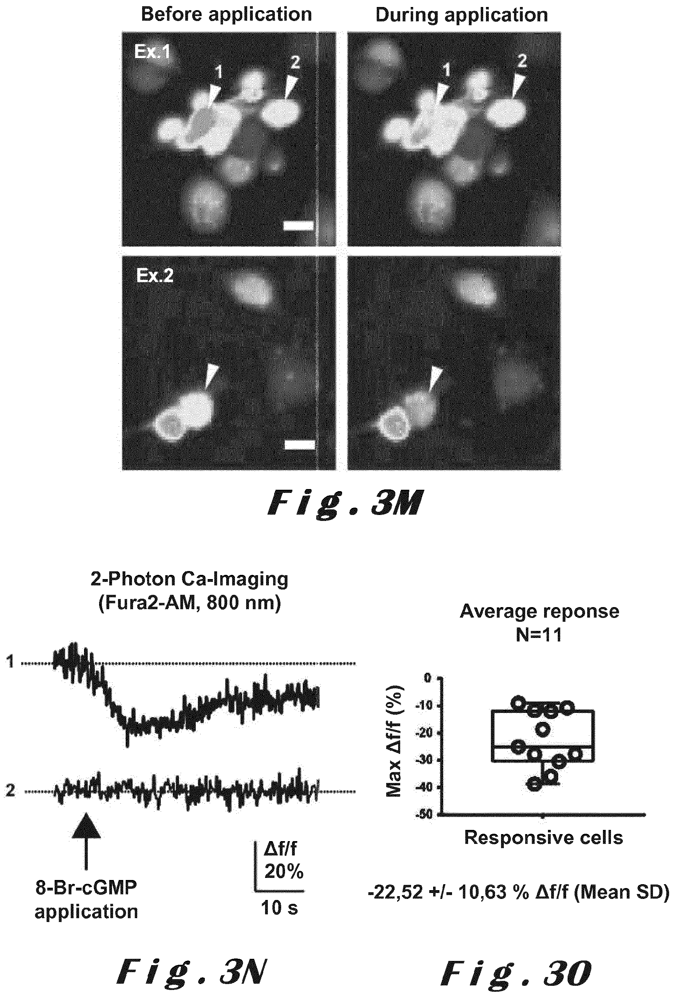

**Figure 3****.** Maturation and electrophysiological analysis of hiPSC-derived photoreceptors.
   (**A-D**) Immunolabeling for RHODOPSIN (A-D), RECOVERIN (A, C) or CONE ARRESTIN (B) and 3DISCO clearing of D195 retinal organoids. Scale bars = 200 µm (A-C) and 25 µm (D). (**E-L**) Transmission electronic microscopy analysis of retinal organoids at D112 and D196 with the presence of external limiting membrane (*), basal bodies (BB), connecting cilia (CC), centrioles (CT) mitochondria (MT), inner segments (IS) and disc stacks (DS) Photoreceptor-specific microtubule arrangements revealed the presence of the clearly identifiable 9x3+0 basal body complex BB and 9x2+0 connecting cilium (CC). Scale bars = 1 µm (E, G, H); 500 nm (F, I, L); 100 nm (J, K). (**M**) Calcium imaging on dissociated retinal cells at D175. Examples (Ex.1 and Ex.2) of 2-Photon Fura2-AM fluorescence images obtained before (left) and during (right) application of 8-Br-cGMP. White arrows on Ex.1 denoted 1 and 2 represent a responsive and a non-responsive cell, respectively. On Ex.2, the arrow represents another responsive cell. Fluorescent images are represented in false colors and are averages of 20 seconds of activity before or during stimulation. A cGMP-based calcium influx is reflected by a decrease in Fura2-AM fluorescence, and such a response is observed in both examples in one cell, without affecting neighbor cells. Scale bars = 5 µm. (**N**) Fluorescence raw traces and effect of an application of cGMP analogue on the cells 1 and 2 displayed on Ex.1, expressed as percentage change from baseline fluorescence (Δf/f %). (**O**) Boxplot representing the average decrease in fluorescence amplitude of 11 responsive cells from three independent samples.

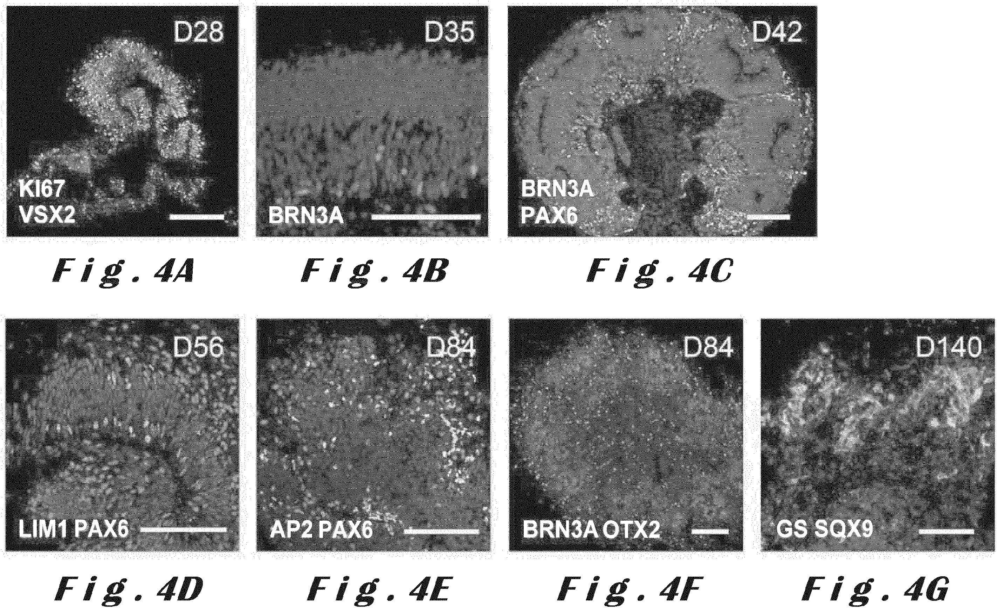

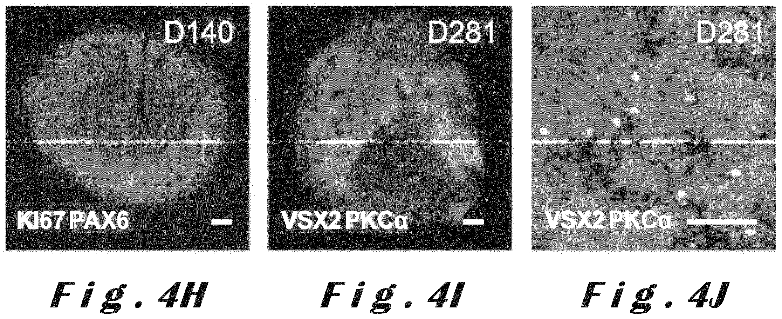

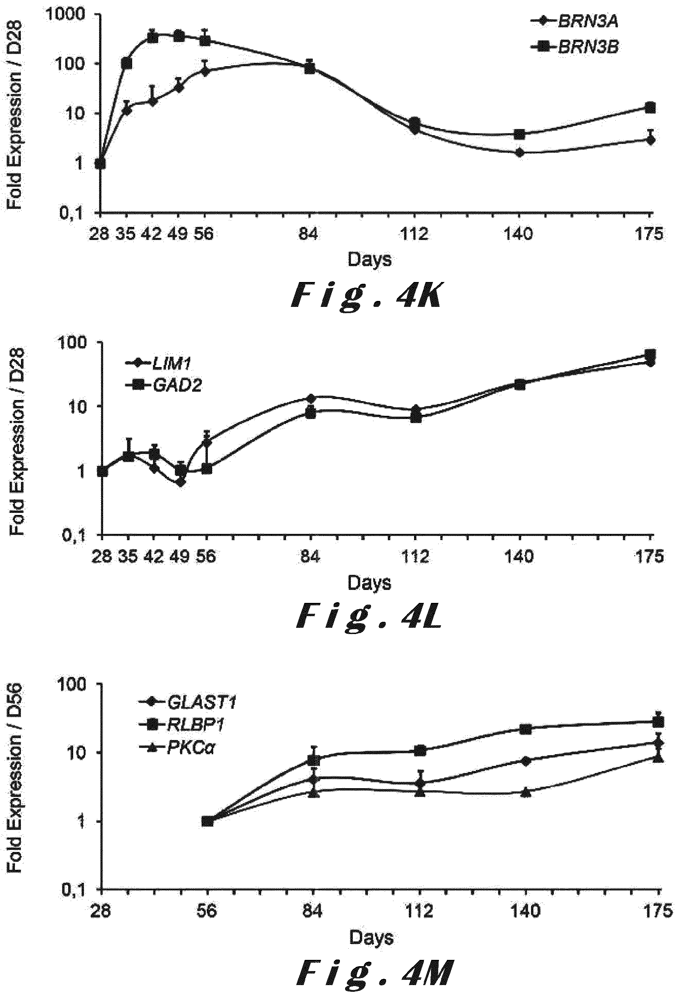

**Figure 4****:** Sequential generation of retinal ganglion cells, horizontal cells, amacrine cells, Müller glial cells and bipolar cells from floating retinal organoids.
   (**A**) Immunofluorescence staining of cryosectioned retinal organoids at D28 for Ki67 and VSX2. (**B-F**) Immunohistochemical analysis of retinal organoids between D35 to D84 using markers for RGCs (BRN3A, PAX6), horizontal cells (LIM1, PAX6), amacrine cells (AP2, PAX6) and photoreceptors (OTX2). Scale bars = 100 µm. (**G-J**) Immunohistochemical analysis of retinal organoids after D140 using markers for proliferating cells (Ki67) and for late retinal cell types, Müller glial cells (GS, SOX9) and bipolar cells (VSX2, PKCα). Nuclei were counterstained with DAPI (blue). Scale bars = 100 µm (H, I) and 50 µm (G, J). (**K-M**) RT-qPCR analysis of selected neural retinal cell types in retinal organoids at different times: RGCs (BRN3A, BRN3B); horizontal cells (LIM), amacrine cells (GAD2), Müller glial cells (GLAST1, RLBP1) and bipolar cells (PKCα). Data are normalized to retinal organoids at D28 (K, L) or D56 (M).

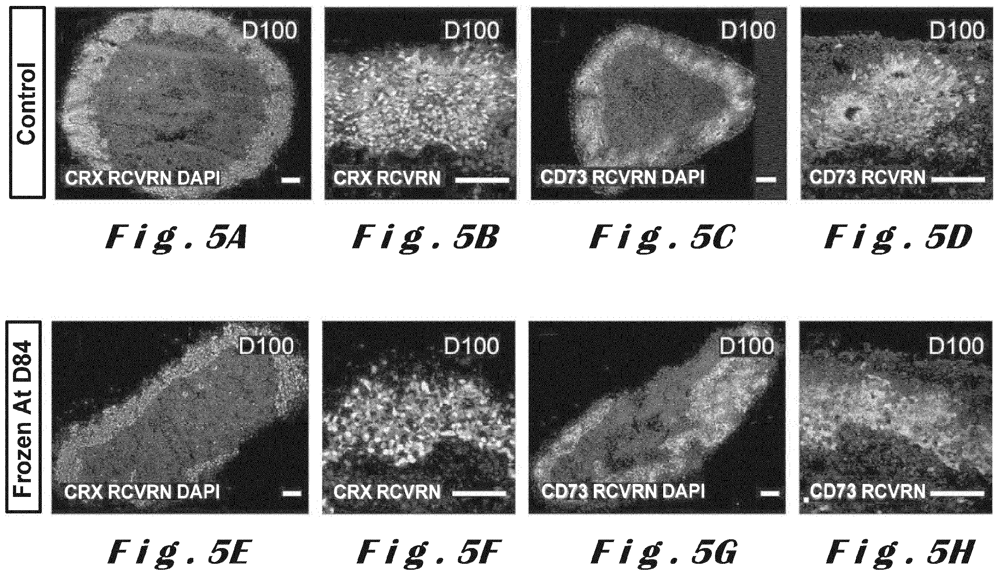

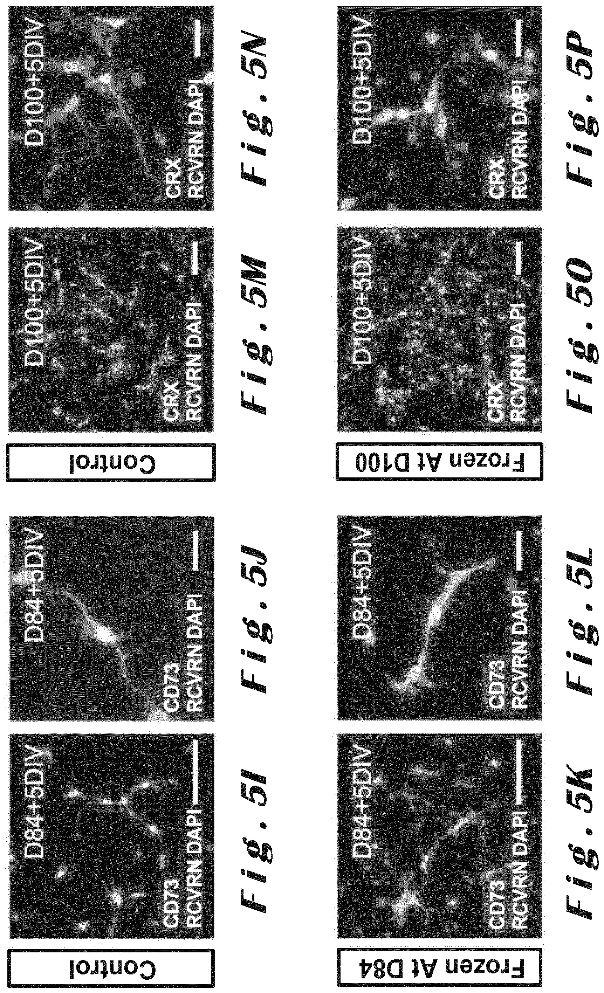

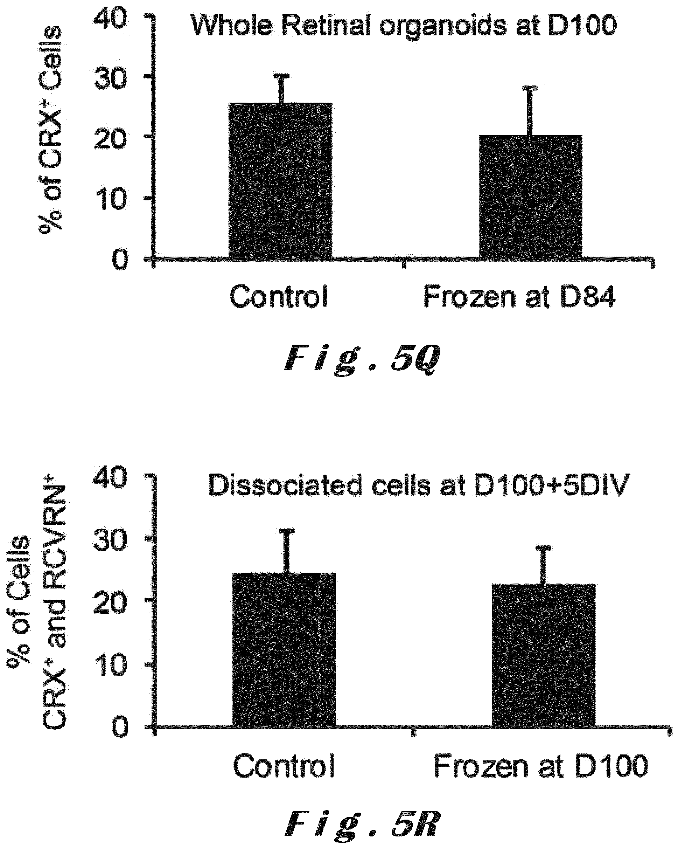

**Figure 5****.** Efficient cryopreservation of photoreceptor precursors in whole retinal organoids or as dissociated cells.
   (**A-D**) Immunostaining for CRX (A, B), CD73 (C, D) and RECOVERIN (A-D) on retinal organoids at D100. Scale bars = 100 µm. (**E-H**) Immunostaining for CRX (E, F), CD73 (G, H) and RECOVERIN (E-H) on retinal organoids cryopreserved at D84 and cultured for additional 16 days after thawing. Scale bars = 100 µm. (**I-P**) Retinal organoids were dissociated at D84 (I-L) or D100 (M-P) and retinal cells were either cultured for 5 days in vitro (DIV) (I, J, M, N) or cryopreserved and cultured for 5 DIV after thawing (K, L, O, P) before immunostaining for CD73 (I-L), CRX (M-P) and RECOVERIN (I-P). Scale bars = 100 µm (I, K, M, O) and 25 µm (J, L, N, P). (**Q**) Quantitative analysis at D100 of CRX-positive cells in unfrozen retinal organoids (control, N= 7 organoid sections corresponding to 81,450 cells) or in retinal organoids cryopreserved at D84 (N =11, organoid sections corresponding to 119,339 cells). (**R**) Quantitative analysis after 5 DIV of double CRX/RECOVERIN-positive cells in fresh (control, n=31,303 cells) or cryopreserved dissociated cell population from D100 retinal organoids (n=13,958 cells).

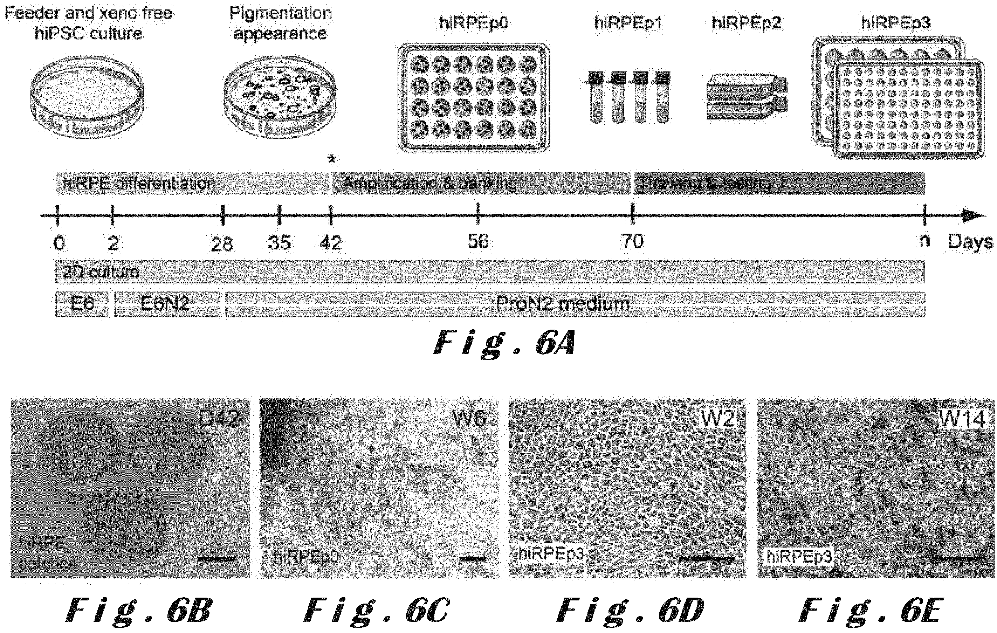

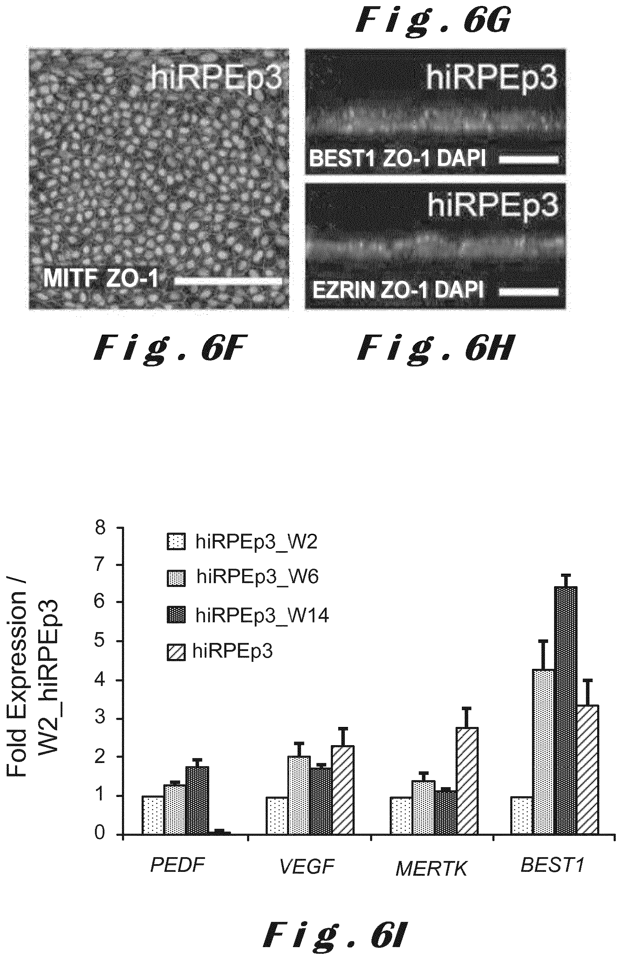

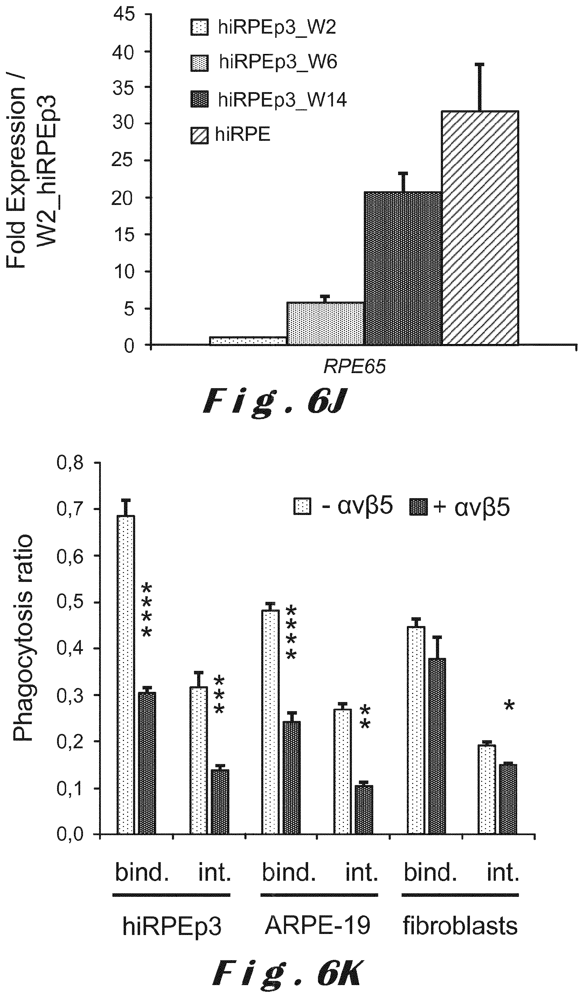

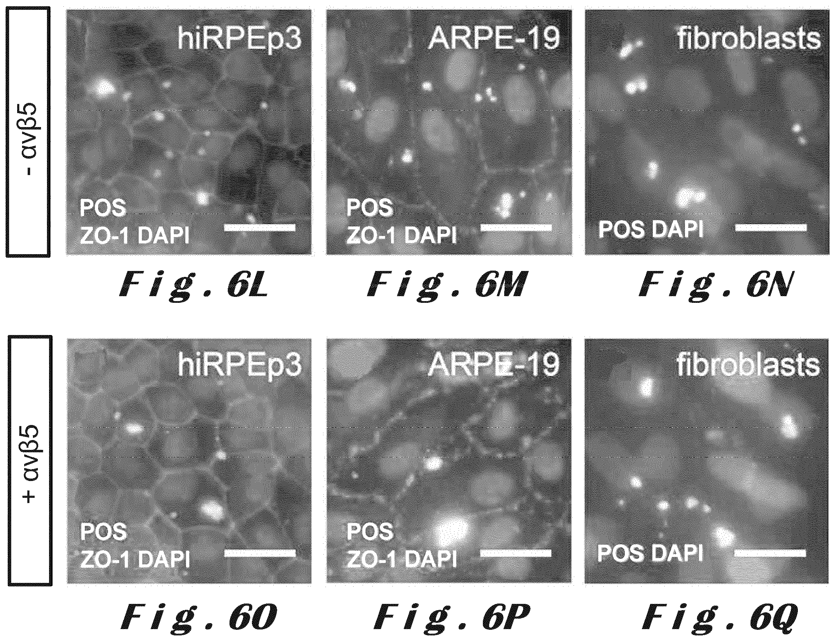

**Figure 6****.** Generation and characterization of hiRPE cells in Xeno-Free and Feeder-Free conditions.
   (**A**) Schematic diagram illustrating different steps for the generation, amplification, and storage of RPE cells from adherent culture of iPSCs. (*) Pigmented patch picking time. (**B**) Phase-contrast images of pigmented patches of hiRPE cells from hiPSC-2 at D42. Scale bar = 3 cm. (**C-E**) Phase-contrast images of hiRPE cells at passage 0 (hiRPEp0) and at passage 3 (hiRPEp3), respectively at 6 weeks (W6) after picking and at W2 or W14 after passaging. Scale bar = 100 µm. (**F**) MITF and ZO-1 immunostaining of hiRPEp3 cells, 2 weeks in culture after thawing. Scale bar = 100 µm. (**G, H**) Z-stack confocal images showing typical polarized expression of BEST1 and ERZIN in hiRPEp3 cells. Scale bars = 10 µm. (**I, J**) RT-qPCR analysis of mature RPE-associated markers in hiRPEp3 cells cultured 2 (W2), 6 (W6) or 14 weeks (W14) after thawing. Data are normalized on hiRPEp3 cells at 2W. (**K**) Evaluation of RPE cell phagocytic activity: ratios of FITC/DAPI fluorescence were evaluated in hiRPEp3 cells, ARPE-19 cell line and fibroblasts after 3hrs of incubation with FITC-labeled POS to determine binding and uptake of POS in absence (blue bars) or in presence (red bars) of function-blocking antibody anti αvβ5 integrin. Values represent the mean ± SD (*: p<0.05; **: p<0.01; ***: p<0.005; ****: p <0.001) from three separate experiments. (**L-Q**). Qualitative representation of internalization of FITC-labeled POS (green) by hiRPEp3, ARPE-19 and fibroblasts (nuclei counterstained in blue) in absence or in presence of the anti αvβ5 integrin blocking antibody. Scale bars = 25 µm.

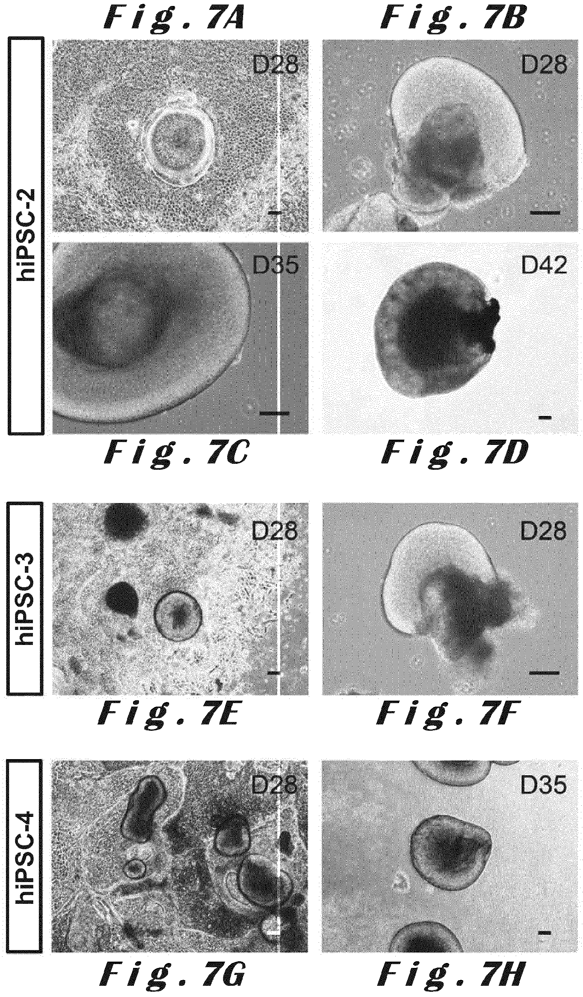

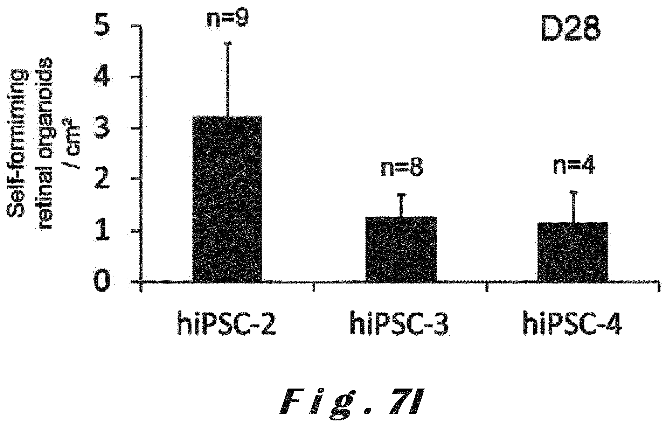

**Figure 7****:** Reproducibility of the retinal organoid formation with different hiPSC lines.

Morphology of the self-forming neuroretina-like structures at D28 (A, E, G) and derived retinal organoids at D28 (B, F), D35 (C, H) and D42 (D), generated from three different integration-free hiPSCs: hiPSC-2 derived from adult dermal fibroblasts reprogrammed by an episomal approach (A-D); hiPSC-3 derived from neonatal foreskin fibroblasts reprogrammed by an episomal approach (E, F) and hiPSC-4 derived from adult dermal fibroblasts reprogrammed by the Sendai virus method (G, H). Scale bars = 100 µm. Efficiency of retinal organoid formation was determined by calculating the number of structures per cm2 at D28 for the three different hiPSC lines (I). The number of independent differentiation experiments is noted for each hiPSC lines.

### EXAMPLES

### Example 1: Reliable and efficient differentiation of human integration-free induced pluripotent stem cells into retinal neurons and retinal pigmented epithelial cells using a feeder-free, xeno-free method.

### 1.1 EXPERIMENTAL PROCEDURES

### Human fibroblast and iPS cell cultures

Established hiPSC-2 clone [19], previously cultured on mitomycin inactivated mouse embryonic fibroblasts, was adapted to feeder-free conditions. iPS colonies from hiPSC-2 clone cells were incubated 7 to 10 min in 2 ml of enzyme-free Gentle cell dissociation reagent (STEMCELL Technologies) at room temperature. After aspiration of the dissociation solution, detached cell aggregates were resuspended in 2 ml of pre-warmed chemical defined Essential 8^{™} medium (Thermo Fischer Scientific) by pipetting up and down the center of the iPS colonies without detaching the feeder cells. Human iPSCs were transferred on truncated recombinant human vitronectin (rhVTN-N)-coated dishes with Essential 8^{™} medium. Cells were routinely cultured at 37°C in a standard 5% CO2 / 95% air incubator with a daily medium change. iPS cells were passaged with the enzyme-free Gentle cell dissociation reagent (2 ml for 7 min at room temperature) every week. Detached cell aggregates were collected in Essential 8TM medium and carefully pipetted up and down to obtain uniform suspension of cell aggregates that are replated at ratio of 1/10 to 1/60 depending on the confluence. Feeder free-adapted hiPSCs were subjected to retinal differentiation after 3 to 5 passages. Adapted at passage 16 (p16), the hiPSC-2 clone was used between p20 to p40 for characterization and differentiation.

### Retinal differentiation and hiPSC-derived retinal cell cultures

Retinal cell differentiation was based on previously established protocol [28] with some modifications. Human iPSCs were expanded to 70-80 % confluence in 6-cm diameter dishes coated with rhVTN-N (Thermo Fischer Scientific) in Essential 8^{™} medium. At this time, defined as day 0 (D0), hiPSCs were cultured in chemical defined Essential 6^{™} medium (Thermo Fischer Scientific). After 2 days, the medium was switched to E6N2 medium composed of Essential 6^{™} medium, 1% CTS^{™} (Cell Therapy Systems) N2 supplement (Thermo Fischer Scientific), 10 units/ml Penicillin and 10 Mg/ml Streptomycin (Thermo Fischer Scientific). The medium was changed every 2-3 days. On D28, identified self-formed retinal organoids were isolated, using a needle, with the surrounding cells and cultured in 6-well-plates (8 to 12 organoids per well) as floating structures in the ProB27 medium supplemented with 10 ng/ml of animal-free recombinant human FGF2 (Peprotech) and half of the medium was changed every 2-3 days. ProB27 medium is composed of chemical defined DMEM:Nutrient Mixture F-12 (DMEM/F12, 1:1, L-Glutamine), 1% MEM non-essential amino acids, 2% CTS^{™} B27 supplement (Thermo Fischer Scientific), 10 units/ml Penicillin and 10 Mg/ml Streptomycin. At D35, FGF2 was removed and half of the "ProB27 medium" was changed every 2-3 days for the next several weeks.

For hiPSC-derived RPE (hiRPE) cell cultures, identified pigmented patches were cut around D42 and transferred, noted as passage 0 (P0), onto plates coated with CTS^{™} CELLStart^{™} (Thermo Fischer Scientific). hiRPE cells were expanded in the ProN2 medium composed of DMEM/F12, 1% MEM non-essential amino acids, 1% CTS^{™} N2 supplement, 10 units/ml Penicillin and 10 Mg/ml Streptomycin; and the medium was changed every 2-3 days. At confluence, hiRPE cells were dissociated using trypsin and replated at 5x10⁴ cells/cm² onto T-25 cm² CTS^{™} CellStart^{™}-coated dishes for amplification before banking.

### Cryopreservation of hiPSC-derived retinal cells

Three to five retinal organoids at D84 were suspended in 250 µl of cold Cryostem freezing medium (Clinisciences) and frozen in a 1.5 ml cryogenic tube (Nunc) placed in isopropanolbased Mr Frosty freezing container (Thermo Fischer Scientific) at -80°C for a minimum of 4 hours. Frozen tubes were kept in a -150°C freezer for long-term storage. HiRPE cells were frozen at passage 1 in Cryostem freezing medium (1.5x10⁶ cells/ 250 µl) using the same method with the freezing container and were placed at -150°C for long-term storage. Frozen retinal structures or hiRPEp1 cells were thawed quickly at 37°C in a water bath and resuspended in prewarmed dedicated media for downstream investigations.

### Retinal organoid immunostaining-clearing and imaging

Retinal organoids were first incubated at RT on a rotating shaker in a solution (PBSGT) of PBS 1X containing 0.2% gelatin, 0.5% Triton X-100 and 0.01% thimerosal (Sigma-Aldrich) for 3 hr. Samples were next transferred to PBSGT containing the selected primary antibodies (data not shown) and placed at 37°C for 3 days, with rotation at 100 rpm. This was followed by six washes for 30 min in PBSGT at RT. Next, samples were incubated with appropriate secondary antibodies conjugated with either Cy3 or Alexa Fluor 594 (Interchim) diluted at 1:600 in PBSGT overnight. After six 30-min washes in PBSGT at RT, samples were stored at 4°C in PBS until the 3D imaging of solvent-cleared organ (3DISCO) clearing procedure [29].

Samples were first dehydrated in a graded series (50%, 80% and 100%) of tetrahydroflurane (Sigma-Aldrich) diluted in H₂O, during 1hr for each step. This was followed by a delipidation in dichloromethane (Sigma-Aldrich) for at least 20 min, and finally, samples were cleared overnight in dibenzylether (Sigma-Aldrich). 3D imaging was performed either with an ultramicroscope (LaVision BioTec) using the ImspectorPro software (LaVision BioTec) or an upright confocal microscope (Olympus FV1000) with a numerical 10X objective for highresolution imaging, as previously described [29]. Images, 3D volume were generated using Imaris x64 software (version 7.6.1, Bitplane).

### 1.2 RESULTS

### Generation of self-forming retinal organoids in Xeno-Free / Feeder-Free conditions from confluent hiPSCs

Human iPSC colonies from the integration-free iPS cell line 2 (hiPSC-2), previously generated by episomal approach [19], were adapted to XF/FF culture system using the truncated recombinant human vitronectin (rhVTN-N) as synthetic substrate and chemical defined Essential 8^{™} medium [21]. Under these new conditions, qRT-PCR revealed that the expression of pluripotency genes in hiPSC-2 is still comparable to that seen in hESCs (data not shown). hiPSC-2 expanded in XF/FF conditions express the pluripotency markersOCT4 and SSEA4 or SOX2 and TRA1-60 (data not shown) and exhibited a normal karyotype (data not shown). At around 70% of confluence, hiPSC colonies cultured in Essential 8^{™} medium were placed in Essential 6^{™} medium (Essential 8^{™} medium without FGF2 and TGFβ) during 2 days to turn off the self-renewal machinery and encourage the spontaneous differentiation. Since it was reported that N2 supplement alone could be sufficient to direct hiPSCs cultured on feeder layers toward retinal fate [19], different chemically defined media containing 1% of CTS^{™} N2 supplement were tested (Figure 1A). In this XF/FF environment, the use of ProN2 medium validated with hiPSCs on feeder layers [19] led regretfully to cell death (data not shown). Nevertheless, a new retinal differentiation medium (E6N2 medium) was developed, corresponding to Essential 6^{™} medium containing 1% of CTS^{™} N2 supplement, allowing the self formation of neuroepithelial-like structures from adherent hiPSCs in 28 days (Figure 1A). In this XF/FF conditions, differentiating iPSCs started within 14 days (D14) to endogenously express the key BMP and WNT antagonists, DKK1 and NOGGIN respectively, essential for neural differentiation (Figure 1B). About four weeks after the initiation of differentiation, self-forming neuroepithelial-like structures appeared in the culture dishes (Figure 1C). RT-qPCR analysis of these isolated structures at D28 (Figure 1D) revealed an eyefield specification with the robust expression of specific markers, such as SIX3, MITF, VSX2, PAX6, RAX and LHX2, while losing the expression of the pluripotency marker POU5F1 (OCT4) (Figure 1E). Immunostaining at D28 showed that these structures comprise a population of mitotic retinal progenitor cells (RPCs) coexpressing VSX2 and Ki67 (Figure 4A). The expression of transcription factors involved in the photoreceptor lineage, such as CRX, NRL and NEUROD1, was also detected as early as D28 (Figure 1E). Following isolation along with surrounding cells at D28, retinal organoids were cultured as floating structures (Figure 1D) in medium containing CTSTM B27 supplement (ProB27 medium) with human FGF2 during 1 week (Figure 1A) to favor differentiation of the neural retina [30]. Spherical organoids increased in size and the distal part of the neuroepithelium became pigmented around D42 (Figure 7B-D). To confirm the reproducibility of the differentiation process, two other hiPSC lines were subjected to the reproducibility of the differentiation process, two other hiPSC lines were subjected to the XF/FF differentiation process. Our results demonstrated that similar retinal organoids can be obtained indifferently from hiPSCs derived from foreskin fibroblasts reprogrammed by an episomal approach (Figure 7E-F) or derived from adult dermal fibroblasts reprogrammed in XF/FF conditions with the Sendai virus (Figure 7G-H). The evaluation of the number of structures that developed per cm² for these three hiPSC lines at D28 revealed some expected inter-cell line variability in term of efficiency (Figure 7I). Transcription factors involved in retinal specification and differentiation were still expressed in retinal organoids during floating cultures in ProB27 medium, with a noticeable increase of RAX, SIX3 and VSX2 expression after D35, while expression of the non-retinal forebrain marker FOXG1 decreased (Figure 1F). At D35, cells forming retinal organoids co-expressed RAX and PAX6 (Figure 1H and 1I) confirming their eye-field identity [31]. At this time, VSX2⁺ cells were predominantly located in the outer part of the developing neuroepithelium (Figure 1J-L), which also expressed also PAX6 (Figure 1J and 1K). MITF+ cells were found mainly in the distal part of the organoids (Figure 1L and 1M), corresponding to RPE cells. At D35, the PAX6⁺/VSX2⁻ cell population is concentrated at the inner part of neuroepithelium (Figure 1J and 1K), corresponding to the first post-mitotic differentiating retinal neurons, which did not express the Ki67 proliferation marker (Figure 1N and 1O). Some cells in the same location were found to be immunoreactive for a specific marker of RGCs, BRN3A (Figure 4B). Nevertheless, at D35, the outer part of retinal organoids still contained proliferative RPCs identified by the co-expression of VSX2 and Ki67 (Figure 1O). Interestingly, RT-qPCR analysis showed that the emergence of photoreceptor precursors can be detected after D42 by up-regulation of NRL, CRX and NEUROD1 expression (Figure 1G), concomitant to the decrease of VSX2 and RAX expression (Figure 1F).

### Differentiation of RPCs from hiPSC-derived retinal organoids

RT-qPCR analysis on organoid RNA extracts showed that RPCs can be committed in to the photoreceptor lineage, with an increase of CRX (Figure 1G), RECOVERIN (RCVRN) and CONE ARRESTIN (CAR) expression (Figure 2A) throughout the floating culture. The expression of genes specific for mature photoreceptors, such as RHODOPSIN (RHO), BLUE or RED/GREEN (R/G) OPSIN (OPS), emerges only after 100 days (Figure 2B). Immature photoreceptors immunoreactive for CRX, OTX2, and RCVRN can be identified at D49 (Figure 2C and 2D), and a stronger expression of these markers was observed at D84 (Figure 2E). Rods and cones can be clearly identified either by RHO or R/G OPS, BLUE OPS and CAR immunostaining in culture kept for a longer period until D281 (around 9 months) (Figure 2G-N). Interestingly, after 40-50 days, differentiating photoreceptors were often found inside rosettes (Figure 2C-G), even though an external cell layer resembling the outer nuclear layer can be observed in some iPSC-derived retinal organoids (Figure 2H, I). The cell surface marker CD73 is expressed specifically in differentiating RCVRN⁺ photoreceptors at different times of differentiation (Figure 2D, F). The expression of CD73 by photoreceptors was confirmed after D140 by the co-expression with RCVRN, CAR, BLUE OPS and R/G OPS (Figure 2J). Conversely, CD73 was not expressed by PAX6+ cells (corresponding to RGCs and horizontal/amacrine cells) located around the rosettes. An efficient photoreceptor differentiation was also observed in retinal organoids derived from the two other hiPSC lines showing cells expressing RCVRN, CAR, CRX, RHO, BLUE OPS and R/G OPS between D77 and D175. In 175-days old retinal organoids, the connecting cilium marker acetylated TUBULIN revealed the existence of very thin structures juxtaposed to RCVRN+ cells (Figure 2I), suggesting the formation of cilia and photoreceptor outer segments (POS). To analyze the spatial distribution of rosettes containing rods and cones, we performed whole-mount immunostaining with photoreceptor specific markers and 3DISCO clearing procedure on D195 retinal organoids. This technology offers the possibility of imaging entire transparent samples without the need of sectioning, when combined with immunohistochemistry [29]. Spatial arrangement of photoreceptors characterized by the expression of RCVRN/RHO and CAR/RHO was observed on 3D-reconstructed images (Figure 3A, B). The precise 3D pattern of rosettes containing RHO⁺ rods or CAR⁺ cones was visualized in the external part of the retinal organoids (Figure 3C). High-resolution confocal imaging of RHO staining on cleared D195 organoids revealed an intense fusiform staining reminiscent of emerging POS in the center of rosettes (Figure 3D). Similar staining was observed with CAR antibody identifying cones in different retinal organoids. TUNEL assay performed on 195-days old organoids demonstrated that photoreceptors identified by RCVRN, RHO or BLUE OPS staining in the rosettes do not seem to undergo apoptosis. Transmission electronic microscopy analysis confirmed the presence of photoreceptor ultra structures, such as basal body and connecting cilium with a photoreceptor-specific microtubule arrangement at D112 (Figure 3E-3K). Some photoreceptors also presented membranous materials, corresponding to developing POS, which were clearly identified at D196 (Figure 3L). To test whether the hiPSC-derived photoreceptors are capable of achieving functional maturation, we tested their ability to exhibit an inward dark current (IDC). The IDC corresponding to an influx of Na⁺ and Ca²⁺ through the activation of cyclic nucleotide-gated (CNG) channels is the result of the increase of the cGMP concentration in photoreceptors. To mimic this "dark state", we used a membrane-permeant cGMP analogue (8-Br-cGMP) to open cationic CNG channels leading to the IDC, as previously described [18]. Ca²⁺ influx was monitored with live two-photon imaging of the intracellular fluorescent Ca²⁺ indicator Fura-2in dissociated cells from retinal organoids at D175. Forty-eight hours after seeding, some Fura-2-loaded retinal cells showed a calcium influx when exposed to 8-Br-cGMP, as observed by decreased intracellular fluorescence (Figure 3M-N). Average of maximum fluorescence variation during recording of 11 responsive photoreceptors is -22.52 +/- 10.63 % (151 cells analyzed, N=4) (Figure 3O). No decrease of the intracellular fluorescence has been observed in retinal cells exposed to AMES puffs instead of the cGMP analogue (150 cells analyzed, N=4). These functional observations support our morphological data that the XF/FF culture conditions reported here allow a certain level of photoreceptor maturation. The RPCs within retinal organoids are able to give rise to other retinal cell types. By RTqPCR and immunostaining using BRN3 markers, we confirmed the early emergence of the RGCs after 35 days of differentiation (Figure 4C and 4K). Interestingly, BRN3A⁺ RGCs localized around the rosettes containing OTX2⁺ photoreceptors can still be observed in 84-days old organoids when ProB27 medium was used (Figure 4F). Similar immunofluorescence analysis confirmed the differentiation of RGCs in retinal organoids derived from the two other hiPSC lines, confirming the reproducibility of the differentiation process. Differentiating amacrine and horizontal cells were detected by RT-qPCR with the induction of GAD2 and LIM expression respectively (Figure 4L) and by immunohistochemistry with the presence of AP2⁺/PAX6⁺ and LIM1⁺/PAX6⁺ cells respectively (Figure 4D and 4E). RT-qPCR demonstrated that bipolar cells identified by PKCα expression appear later (Figure 4M) and immunostaining at D281 confirmed the presence of fully differentiated bipolar cells co-expressing VSX2 and PKCα (Figure 4I, J). The emergence of Müller glial cells was also observed at later time points, as shown by RT-qPCR with the induction of specific markers GLAST1 and RLBP1 (Figure 4M) and by the identification of cells co-expressing the GLUTAMINE SYNTHETASE (GS) and SOX9 (Figure 4G). At D140, the presence of very rare Ki67+ cells (less than 5 positive cells per section) indicates that retinal organoids reached a "mature state", confirmed by the absence of mitotic RPCs (Figure 4H).

### Cryopreservation of photoreceptor precursors from retinal organoids

Considering the potential use of photoreceptor precursors for future cell transplantation [32, 33] and the length of the differentiation, we sought to develop different cryopreservation approaches that would enable the cells to be stored along the differentiation process. The first strategy was to cryopreserve whole retinal organoids at stages of development where CD73 is well expressed (close to D100, as shown by immunofluorescence). Retinal organoids cultured in suspension until D84 were submitted to cryopreservation using cold Cryostem^{®} freezing medium and the presence of photoreceptors was examined in organoids 16 days after thawing, at D100, in floating cultures. Frozen retinal organoids showed the presence of intact rosettes containing CRX⁺ and RCVRN⁺ photoreceptor precursors (Figure 5E, F) similar to non-frozen organoids at the same stage (Figure 5A, B). Interestingly, the CD73⁺ cells, considered as the transplantable cell population [34-36], were still clearly identified. Indeed, as in non-frozen organoids (Figure 5C, D), CD73 is specifically expressed in RCVRN⁺ cells (Figure 5G, H) confirming their specific expression in photoreceptors. Quantification of CRX⁺ cells at D100 (Figure 5Q) did not reveal any significant difference between the number of cells in controls (non frozen) and frozen retinal organoids (p=0.1344; n=81,450 and 119,339 counted cells). In a second approach, we examined whether dissociated retinal cells from organoids could be cryopreserved. In this case, retinal organoids cultured until D84 or D100 were dissociated using papain and retinal cells were cryopreserved using the cold Cryostem^{®} freezing medium. After thawing, cells were plated on Poly-D-Lysin / Laminin treated coverslips to be cultured for 5 additional days in vitro (DIV). Immunostaining revealed that frozen RCVRN⁺ photoreceptors have kept their strong CD73 expression as in non frozen dissociated cells (Figure 5I-L). Similar observations were made on a double CRX⁺ and RCVRN⁺ cell population (Figure 5M-P). Accordingly, quantification showed no significant difference in the number of double CRX⁺ and RCVRN⁺ cells was observed between frozen cells and control cells (Figure 5R) (p=0.5839; n=31,303 and 23,958 counted cells).

### Generation, amplification and banking of hiPSC-derived RPE cells.

Human iPS-derived RPE (hiRPE) cells were generated simultaneously to the retinal organoids from confluent hiPSCs cultured in Essential 6^{™} medium with the successive addition after 2 days of the CTSTM N2 supplement until D28 (Figure 6A). After removal of self-forming retinal organoids, cell cultures were switched to ProN2 medium. Around D42, pigmented patches of cells were picked up and hiRPE cells were amplified from these patches (Figure 6B, C). After 2 weeks, hiRPE cells at passage 1 (hiRPEp1) were cryopreserved for cell banking and thawed hiRPE cells at passage 3 (hiRPEp3) were used for complete characterization (Figure 6D, E). Generated epithelial cells were immunoreactive for the key RPE-specific transcription factor MITF and the tight junction marker ZO-1 (Figure 6F). Counting the number of MITF+ cells confirmed the purity of the cell culture with 99.83 ± 0.31 % of positive cells (n=208,259 cells). Immunostaining for BESTROPHIN (BEST1) and EZRIN showed a correct polarization of hiRPE cells in culture with the expression of these proteins at the basolateral membrane and the apical side of the cells respectively (Figure 6G, H). RT-qPCR analysis also demonstrates that cells retained the expression of mature RPE-associated markers such as *PEDF*, *VEGF*, *MERTK* and *BEST1* after freezing and at least for 3 additional passages (Figure 6I). Long-term cultures of hiRPEp3 cells depicted a closer RPE phenotype to human primary cultures, particularly for *RPE65*, which is strongly expressed in adult human RPE cells (Figure 6J). We investigated whether hiRPE cells after banking still exhibited typical native RPE functions. Using ELISA we showed vascular endothelial growth factor (VEGF) and pigment epithelium derived factor (PEDF) secretion by hiRPEp3 cells with respective values of 12.4+0.5 ng/ml and 80.5+8.8 ng/ml (n=3). We also demonstrated the ability of hiRPEp3 cells to carry out specific phagocytosis of FITC-labeled POS similarly to the human immortalized RPE cell line ARPE-19 (Figure 6K-Q). Conversely to fibroblasts, POS phagocytosis by both hiRPE and ARPE-19 cells was blocked by an antibody against integrin αvβ5, demonstrating the specificity of POS phagocytosis linked to this receptor by RPE cells, as previously demonstrated [37, 38].

In the current study, we propose a new protocol to generate retinal organoids and RPE cells from adherent hiPSCs using a defined XF/FF culture system amenable to future clinical grade production. To keep simple a protocol avoiding EB formation and the use of substrates such as Matrigel as previously described in xenogeneic condition [19, 28], retinal differentiation in the XF/FF culture conditions has required the assessment of critical points. We have determined the right time-point to start the differentiation of adherent hiPSCs, developed new chemical defined differentiation medium and established the optimal timing to collect emerging retinal organoids. The replacement of feeders by human vitronectin, which supported pluripotency of hESCs and hiPSCs in a XF defined medium [21], enables the generation of self-forming neuroretinal structures and RPE cells, when expanded iPSC colonies were placed in successive chemically-defined proneural media with animal-free CTS^{™} supplements (ThermoFischer Scientific). The endogenous production by hiPSCs of DKK1 and NOGGIN, two inducers of neural and retinal specification, could explain the self-formation of these structures, as previously reported [19]. The presence of recombinant human Insulin in the CTS^{™} N2 supplement, could play a similar role to that of IGF-1, generally added to promote the retinal lineage [9, 12]. Isolated structures cultured as floating retinal organoids in ProB27 medium allowed the differentiation of multipotent RPCs in all the retinal cell types in a sequential overlapping order, similar to the one we previously reported in xenogeneic conditions [19]. RGCs were detected as early as D35 followed by horizontal and amacrine cells, whereas mature photoreceptors, expressing OPSIN or RHODOPSIN, and bipolar or Müller glial cells were clearly identified after a D100. Interestingly, our culture conditions allowed the maintenance of both mature S and L/M cones and rods in retinal organoids until D281 (>9 months). The presence of a specific PR ultrastructures confirms that our XF/FF conditions permit the maturation of photoreceptors with similar structures to those observed in the developing human retina [39]. Further evidence of the advanced level of maturation is the sensitivity of a proportion of hiPSC-derived photoreceptors to cGMP stimulation in a similar manner to native photoreceptors. Moreover, we demonstrated that adaptation of the tissue clearing procedure 3DISCO [29] to retinal organoids, allowed the analysis of fluorescently labeled retinal cells in transparent organoids while preserving original shape and structure. This approach provides a global view of retinal cell type development, spatial arrangement and connections within the organoids. When combined with high-resolution microscopy, this technique could be used for a rapid and efficient analysis of normal and pathological features in intact retinal organoids generated from patient-specific iPSCs.

For future cell therapy strategies based on purified photoreceptor precursors, the method of the invention allows, in less than 100 days, the generation of CD73-positive photoreceptor precursors, described as a transplantation-compatible cell population in mouse [34-36]. We have demonstrated that CD73 is a marker of photoreceptor precursors and continues to be expressed in differentiated photoreceptors, particularly cones expressing R/G or BLUE OPSIN, in contrast to mice where rare BLUE OPSIN cones do not express CD73 [40]. Since the transplantation in ONL-degenerated host retinas should also be considered, the intact neuroretinal tissue of the retinal organoids derived from hiPSCs in XF/FF conditions could be of clinical utility, as recently proposed for hESC-derived retinal tissues [41]. In this context of retinal sheet transplantation, the ontogenic stages of differentiated tissues from hESCs or hiPSCs should be clearly defined for successful transplantation. Nevertheless, a therapeutic translation of these cells would require a constant production in terms of cell quantity and consistency that it is not likely to be achieved with a fresh cell therapy product (CTP). Therefore, developing an adequate cryopreservation of the CTP is a crucial aspect to ensure a continual delivery of the cell population of interest. Cryopreservation of the whole retinal organoids demonstrated that in frozen-thawed structures the CD73⁺ photoreceptor precursor population is preserved. This should make it possible to prepare large-scale stocks of retinal organoids at specific stages to deliver pure transplantation-competent CD73⁺ cells when needed. Furthermore, our freezing method enables the cryopreservation of dissociated retinal cells including photoreceptor precursors without loss of membrane expression of CD73. This observation highlights the alternative possibility of using thawed dissociated retinal cells and potentially a thawed and homogenous population of CD73⁺ cells for cell transplantation. However, it will be important to determine what kind of cryopreservation process and purification method induces less cell stress and would be the most effective for subretinal grafting.

In addition to photoreceptor precursors, the production of hiPSC-derived RGCs in our XF/FF conditions could have important implications for the treatment of retinal dystrophies affecting the RGCs such as glaucoma. The fast emergence of RGCs in organoids and the use of CTS^{™} B27-containing medium that keeps them alive longer compared to N2-containing medium [19], should facilitate the development of cell-based therapy approaches using hiPSC-derived RGCs. Our cryopreservation technique would be useful to freeze retinal organoids at relatively early stages (before day 50) to make frozen stocks of RGC precursors that could be deliver when needed.

Concomitantly to the neural retina, our protocol allows the generation of RPE cells from hiPSCs that can be easily amplified, passaged and frozen while retaining a proper RPE phenotype and RPE specific functions such as POS phagocytosis and trophic factor secretion. Gene expression assays indicated that hiRPE cell maturation could be achieved by extending the length of culture time. Large-scale production of hiRPE cells can be reached with our XF/FF conditions, since one 6-cm² dish can produce up to 500 millions of hiRPE cells after 3 passages. Based on previous reports, using either hESC-derived RPE cells in suspension [25] or sheets of hiPSC-derived RPE cells [42], we believe that the XF/FF differentiation protocol reported here could deliver sufficient quantities of cells to perform several thousands of injections. A large-scale production of these cells could be required with the future development of global haplotype hiPSC banking, where a limited number of cell lines with common HLA haplotypes will be derived in order to match a significant portion of the patient population [43, 44].

The data shows that simple and efficient retinal differentiation of adherent hiPSCs can be accomplished with the methods of the invention in XF/FF environment near to clinical conditions with the use of minimal and GMP-compatible raw materials. The methods of the invention, illustrated by the above example, is amenable to the development of an in vitro GMP-compliant retinal cell manufacturing protocol allowing for large-scale production and banking of hiPSC-derived retinal cells and tissues. Human iPSCs used in this data have been generated by a non-integrative approach, thus addressing one major limitation for future clinical applications of hiPSCs generated from integrative delivery systems.

### REFERENCES

1. Osakada F, Ikeda H, Mandai M, et al. Toward the generation of rod and cone photoreceptors from mouse, monkey and human embryonic stem cells. Nat. Biotechnol. 2008;26(2):215-24.
2. Idelson M, Alper R, Obolensky A, et al. Directed differentiation of human embryonic stem cells into functional retinal pigment epithelium cells. Cell Stem Cell 2009;5(4):396-408.
3. Krohne TU, Westenskow PD, Kurihara T, et al. Generation of retinal pigment epithelial cells from small molecules and OCT4 reprogrammed human induced pluripotent stem cells. Stem Cells Transl. Med. 2012;1(2):96-109.
4. Kokkinaki M, Sahibzada N, Golestaneh N. Human induced pluripotent stem-derived retinal pigment epithelium (RPE) cells exhibit ion transport, membrane potential, polarized vascular endothelial growth factor secretion, and gene expression pattern similar to native RPE. Stem Cells 2011;29(5):825-35.
5. Zahabi A, Shahbazi E, Ahmadieh H, et al. A new efficient protocol for directed differentiation of retinal pigmented epithelial cells from normal and retinal disease induced pluripotent stem cells. Stem Cells Dev. 2012;21(12):2262-72.
6. Buchholz DE, Pennington BO, Croze RH, et al. Rapid and efficient directed differentiation of human pluripotent stem cells into retinal pigmented epithelium. Stem Cells Transl. Med. 2013;2(5):384-93.
7. Maruotti J, Wahlin K, Gorrell D, et al. A simple and scalable process for the differentiation of retinal pigment epithelium from human pluripotent stem cells. Stem Cells Transl. Med. 2013;2(5):341-54.
8. Meyer JS, Shearer RL, Capowski EE, et al. Modeling early retinal development with human embryonic and induced pluripotent stem cells. Proc. Natl. Acad. Sci. U. S. A. 2009; 106(39): 16698-703.
9. Zhu Y, Carido M, Meinhardt A, et al. Three-dimensional neuroepithelial culture from human embryonic stem cells and its use for quantitative conversion to retinal pigment epithelium. PLoS One 2013;8(1):e54552.
10. Zhou S, Flamier A, Abdouh M, et al. Differentiation of human embryonic stem cells into cone photoreceptors through simultaneous inhibition of BMP, TGFβ and Wnt signaling. Development 2015;142(19):3294-306.
11. Yanai A, Laver CRJ, Joe AW, et al. Differentiation of human embryonic stem cells using size-controlled embryoid bodies and negative cell selection in the production of photoreceptor precursor cells. Tissue Eng. Part C. Methods 2013;19(10):755-64.
12. Lamba DA, Karl MO, Ware CB, et al. Efficient generation of retinal progenitor cells from human embryonic stem cells. Proc. Natl. Acad. Sci. U. S. A. 2006;103(34):12769-74.
13. Tucker BA, Mullins RF, Streb LM, et al. Patient-specific iPSC-derived photoreceptor precursor cells as a means to investigate retinitis pigmentosa. Elife 2013;2:e00824.
14. Mellough CB, Sernagor E, Moreno-Gimeno I, et al. Efficient stage-specific differentiation of human pluripotent stem cells toward retinal photoreceptor cells. Stem Cells 2012;30(4):673-86.
15. Meyer JS, Howden SE, Wallace KA, et al. Optic vesicle-like structures derived from human pluripotent stem cells facilitate a customized approach to retinal disease treatment. Stem Cells 2011;29:1206-1218.
16. Nakano T, Ando S, Takata N, et al. Self-formation of optic cups and storable stratified neural retina from human ESCs. Cell Stem Cell 2012;10(6):771-85.
17. Zhong X, Gutierrez C, Xue T, et al. Generation of three-dimensional retinal tissue with functional photoreceptors from human iPSCs. Nat. Commun. 2014;5(May):4047.
18. Mellough CB, Collin J, Khazim M, et al. IGF-1 Signalling Plays an Important Role in the Formation of Three Dimensional Laminated Neural Retina and Other Ocular Structures from Human Embryonic Stem Cells. Stem Cells 2015;33(8):2416-30.
19. Reichman S, Terray A, Slembrouck A, et al. From confluent human iPS cells to self-forming neural retina and retinal pigmented epithelium. Proc. Natl. Acad. Sci. U. S. A. 2014;111(23):8518-23.
20. Singh RK, Mallela RK, Cornuet PK, et al. Characterization of Three-Dimensional Retinal Tissue Derived from Human Embryonic Stem Cells in Adherent Monolayer Cultures. Stem Cells Dev. 2015;24(23):2778-95.
21. Chen G, Gulbranson DR, Hou Z, et al. Chemically defined conditions for human iPSC derivation and culture. Nat. Methods 2011;8(5):424-429.
22. Vaajasaari H, Ilmarinen T, Juuti-Uusitalo K, et al. Toward the defined and xeno-free differentiation of functional human pluripotent stem cell-derived retinal pigment epithelial cells. Mol. Vis. 2011;17:558-75.
23. Pennington BO, Clegg DO, Melkoumian ZK, et al. Defined culture of human embryonic stem cells and xeno-free derivation of retinal pigmented epithelial cells on a novel, synthetic substrate. Stem Cells Transl. Med. 2015;4(2):165-77.
24. Plaza Reyes A, Petrus-Reurer S, Antonsson L, et al. Xeno-Free and Defined Human Embryonic Stem Cell-Derived Retinal Pigment Epithelial Cells Functionally Integrate in a Large-Eyed Preclinical Model. Stem Cell Reports 2015;6:1-9.
25. Schwartz SD, Tan G, Hosseini H, et al. Subretinal Transplantation of Embryonic Stem Cell-Derived Retinal Pigment Epithelium for the Treatment of Macular Degeneration: An Assessment at 4 Years. Invest. Ophthalmol. Vis. Sci. 2016;57(5):ORSFc1-9.
26. Tucker BA, Anfinson KR, Mullins RF, et al. Use of a synthetic xeno-free culture substrate for induced pluripotent stem cell induction and retinal differentiation. Stem Cells Transl. Med. 2013;2(1):16-24.
27. Sridhar A, Steward MM, Meyer JS. Nonxenogeneic growth and retinal differentiation of human induced pluripotent stem cells. Stem Cells Transl. Med. 2013;2(4):255-64.
28. Reichman S, Goureau O. Production of Retinal Cells from Confluent Human iPS Cells. Methods Mol. Biol. 2016;1357:339-51.
29. Belle M, Godefroy D, Dominici C, et al. A Simple Method for 3D Analysis of Immunolabeled Axonal Tracts in a Transparent Nervous System. Cell Rep. 2014;9(4):1191-1201.
30. Fuhrmann S. Eye morphogenesis and patterning of the optic vesicle. Curr. Top. Dev. Biol. 2010;93:61-84.
31. Mathers PH, Jamrich M. Regulation of eye formation by the Rx and pax6 homeobox genes. Cell. Mol. Life Sci. 2000;57(2):186-94.
32. MacLaren RE, Pearson RA, MacNeil A, et al. Retinal repair by transplantation of photoreceptor precursors. Nature 2006;444(7116):203-7.
33. Barber AC, Hippert C, Duran Y, et al. Repair of the degenerate retina by photoreceptor transplantation. Proc. Natl. Acad. Sci. U. S. A. 2013;110(1):354-9.
34. Lakowski J, Han Y-T, Pearson RA, et al. Effective transplantation of photoreceptor precursor cells selected via cell surface antigen expression. Stem Cells 2011;29(9):1391-404.
35. Eberle D, Schubert S, Postel K, et al. Increased integration of transplanted CD73-positive photoreceptor precursors into adult mouse retina. Invest. Ophthalmol. Vis. Sci. 2011;52(9):6462-71.
36. Ferreira TS, Postel K, Stutzki H, et al. Daylight Vision Repair by Cell Transplantation. 2014;(1):1-15.
37. Finnemann SC, Bonilha VL, Marmorstein AD, et al. Phagocytosis of rod outer segments by retinal pigment epithelial cells requires alpha(v)beta5 integrin for binding but not for internalization. Proc. Natl. Acad. Sci. U. S. A. 1997;94(24):12932-7.
38. Nandrot EF, Anand M, Almeida D, et al. Essential role for MFG-E8 as ligand for alphavbeta5 integrin in diurnal retinal phagocytosis. Proc. Natl. Acad. Sci. U. S. A. 2007; 104(29): 12005-12010.
39. Hollenberg MJ, Spira AW. Human retinal development: Ultrastructure of the outer retina. Am. J. Anat. 1973;137(4):357-385.
40. Koso H, Minami C, Tabata Y, et al. CD73, a novel cell surface antigen that characterizes retinal photoreceptor precursor cells. Invest. Ophthalmol. Vis. Sci. 2009;50(11):5411-8.
41. Shirai H, Mandai M, Matsushita K, et al. Transplantation of human embryonic stem cell-derived retinal tissue in two primate models of retinal degeneration. Proc. Natl. Acad. Sci. 2016;113(1):201512590.
42. Kamao H, Mandai M, Okamoto S, et al. Characterization of human induced pluripotent stem cell-derived retinal pigment epithelium cell sheets aiming for clinical application. Stem Cell Reports 2014;2(2):205-218.
43. Taylor CJ, Peacock S, Chaudhry AN, et al. Synthesis Generating an iPSC Bank for HLA-Matched Tissue Transplantation Based on Known Donor and Recipient HLA Types. 2012:147-152.
44. Wilmut I, Leslie S, Martin NG, et al. Development of a global network of induced pluripotent stem cell haplobanks. Regen. Med. 2015;10(3):235-8.
45.Chung Y et al, Human Embryonic Stem Cell Lines Generated without Embryo Destruction, Cell Stem Cell; 2008, 2(2):113-117,).

## Claims

1. An *in vitro* method for obtaining human retinal progenitors, comprising the steps of:
(i) placing an adherent culture of human pluripotent stem cells into a stem-cell-specific pro-neural medium. said culture being devoid of feeder cells; and
(ii) maintaining this culture in said stem-cell-specific pro-neural medium until the appearance of pigmented cells and/or of neuroepithelial-like structures,
said stem-cell-specific pro-neural medium comprising a nutrient cell medium consisting in insulin, selenium, transferrin, L-ascorbic acid, in DMEM/F12 with pH adjusted with NaHCO₃ and a supplement which comprises at least part of the following elements: carbon sources, vitamins, inorganic salts, amino acids and a protein digest.

2. The method of claim 1, wherein said stem-cell-specific pro-neural medium further comprises TGFβ and/or FGF2.

3. The method of claim 1 or 2, wherein said stem-cell-specific pro-neural medium comprises or consists of progesterone, putrescine, sodium selenite, insulin, selenium, transferrin, L-ascorbic acid, in DMEM/F12 with pH adjusted with NaHCO₃, preferably wherein all components of the stem-cell-specific pro-neural medium are derived from the same organism, advantageously derived from human origin.

4. The method of any of the preceding claims, wherein step (ii) is performed until the adherent culture reaches 60-80%, advantageously 70-80% confluence.

5. The method of any of the preceding claims, wherein step (ii) is performed during at least 16 days, preferably between 19 to 33 days.

6. The method of any of the preceding claims, for obtaining retinal pigmented epithelial cells (RPE cells), wherein said method further comprises the steps of
(iii_{RPE}) collecting, from the culture obtained in step (ii), at least one pigmented cell; and
(iv_{RPE}) culturing the pigmented cell(s) obtained in step (iii_{RPE}).

7. The method of claim 6, wherein the culture in step (iv_{RPE}) is carried out in an adherent culture system.

8. The method of any of claims 1 to 7, for obtaining neural retinal cells, wherein said method further comprises the steps of:
(iii_{NR}) collecting, from the culture obtained in step (ii), cells from at least one neuroepithelial-like structure; and
(iv_{NR}) culturing the cells obtained in step (iii_{NR}).

9. The method of claim 8, wherein at least one neuroepithelial-like structure is collected in step (iii_{NR}).

10. The method of any of claims 8 to 9, wherein in step (iv_{NR}), the culture medium is supplemented with FGF2 during at least 5 days.

## Patentansprüche

1. *In-vitro-* Verfahren zum Erhalten humaner retinaler Vorläufer, umfassend die folgenden Schritte:
(i) Einbringen einer adhärenten Kultur humaner pluripotenter Stammzellen in ein stammzellspezifisches pro-neurales Medium, wobei die Kultur frei von Fütterzellen ist; und
(ii) Halten der Kultur in dem genannten stammzellspezifischen pro-neuralen Medium, bis pigmentierte Zellen und/oder neuroepithelähnliche Strukturen auftreten,
wobei das stammzellspezifische pro-neurale Medium ein Zellnährmedium umfasst, das aus Insulin, Selen, Transferrin und L-Ascorbinsäure, in DMEM/F12, dessen pH-Wert mit NaHCO₃ eingestellt wurde, besteht, sowie einen Zusatz, der mindestens einen Teil der folgenden Elemente umfasst: Kohlenstoffquellen, Vitamine, anorganische Salze, Aminosäuren und ein Proteindigest.

2. Verfahren nach Anspruch 1, wobei das stammzellspezifische pro-neurale Medium weiter TGFβ und/oder FGF2 umfasst.

3. Verfahren nach Anspruch 1 oder 2, wobei das stammzellspezifische pro-neurale Medium Progesteron, Putrescin, Natriumselenit, Insulin, Selen, Transferrin, L-Ascorbinsäure, in DMEM/F12, dessen pH-Wert mit NaHCO₃ eingestellt ist, umfasst oder daraus besteht, wobei vorzugsweise alle Bestandteile des stammzellspezifischen pro-neuralen Mediums aus demselben Organismus stammen, vorteilhafterweise aus menschlichem Ursprung stammen.

4. Verfahren nach einem der vorstehenden Ansprüche, wobei Schritt (ii) so lange durchgeführt wird, bis die adhärente Kultur eine Konfluenz von 60-80 %, vorteilhafterweise 70-80 %, erreicht hat.

5. Verfahren nach einem der vorstehenden Ansprüche, wobei Schritt (ii) über einen Zeitraum von mindestens 16 Tagen, vorzugsweise zwischen 19 und 33 Tagen, durchgeführt wird.

6. Verfahren nach einem der vorstehenden Ansprüche zum Erhalten von retinalen Pigmentepithelzellen (RPE-Zellen), wobei das Verfahren weiter die folgenden Schritte umfasst
(iii_{RPE}) Entnehmen von mindestens einer pigmentierten Zelle aus der in Schritt (ii) erhaltenen Kultur; und
(iv_{RPE}) Kultivieren der in Schritt (iii_{RPE}) erhaltenen pigmentierten Zelle(n).

7. Verfahren nach Anspruch 6, wobei die Kultivierung in Schritt (iv_{RPE}) in einem adhärenten Kultursystem ausgeführt wird.

8. Verfahren nach einem der Ansprüche 1 bis 7 zum Erhalten neuronaler retinaler Zellen, wobei das Verfahren weiter die folgenden Schritte umfasst:
(iii_{NR}) Entnehmen von Zellen aus mindestens einer neuroepithelartigen Struktur aus der in Schritt (ii) erhaltenen Kultur; und
(iv_{NR}) Kultivieren der in Schritt (iii_{NR}) erhaltenen Zellen.

9. Verfahren nach Anspruch 8, wobei in Schritt (iii_{NR}) mindestens eine neuroepithelartige Struktur entnommen wird.

10. Verfahren nach einem der Ansprüche 8 bis 9, wobei in Schritt (iv_{NR}) das Kulturmedium mindestens 5 Tage lang mit FGF2 supplementiert wird.

## Revendications

1. Procédé *in vitro* pour l'obtention de progéniteurs rétiniens humains, comprenant les étapes suivantes :
(i) placer une culture adhérente de cellules souches pluripotentes humaines dans un milieu pro-neural spécifique aux cellules souches, ladite culture étant dépourvue de cellules nourricières ; et
(ii) maintenir cette culture dans ledit milieu pro-neural spécifique aux cellules souches jusqu'à l'apparition de cellules pigmentées et/ou de structures de type neuroépithélial,
ledit milieu pro-neural spécifique aux cellules souches comprenant un milieu cellulaire nutritif constitué d'insuline, de sélénium, de transferrine, d'acide L-ascorbique, dans du DMEM/F12 dont le pH est ajusté avec du NaHCO₃ et d'un supplément qui comprend au moins une partie des éléments suivants : des sources de carbone, des vitamines, des sels inorganiques, des acides aminés et un digestat de protéines.

2. Procédé selon la revendication 1, dans lequel ledit milieu pro-neural spécifique aux cellules souches comprend en outre du TGFβ et/ou du FGF2.

3. Procédé selon la revendication 1 ou 2, dans lequel ledit milieu pro-neural spécifique aux cellules souches comprend ou consiste en de la progestérone, de la putrescine, de sélénite de sodium, d'insuline, de sélénium, de transferrine, d'acide L-ascorbique, dans du DMEM/F12 dont le pH est ajusté avec du NaHCO₃, de préférence dans lequel tous les composants du milieu pro-neural spécifique aux cellules souches sont dérivés du même organisme, de préférence d'origine humaine.

4. Procédé selon l'une quelconque des revendications précédentes, dans lequel l'étape (ii) est réalisée jusqu'à ce que la culture adhérente atteigne une confluence de 60 à 80 %, de préférence de 70 à 80 %.

5. Procédé selon l'une quelconque des revendications précédentes, dans lequel l'étape (ii) est réalisée pendant au moins 16 jours, de préférence entre 19 et 33 jours.

6. Procédé selon l'une quelconque des revendications précédentes, pour obtenir des cellules épithéliales pigmentées de la rétine (cellules EPR), dans lequel ledit procédé comprend en outre les étapes consistant à
(iii_{RPE}) prélever, à partir de la culture obtenue à l'étape (ii), au moins une cellule pigmentée ; et
(iv_{RPE}) cultiver la ou les cellules pigmentées obtenues à l'étape (iii_{RPE}).

7. Procédé selon la revendication 6, dans lequel la culture de l'étape (iv_{RPE}) est réalisée dans un système de culture adhérente.

8. Procédé selon l'une quelconque des revendications 1 à 7, pour obtenir des cellules rétiniennes neurales, dans lequel ledit procédé comprend en outre les étapes consistant à :
(iii_{NR}) prélever, à partir de la culture obtenue à l'étape (ii), des cellules provenant d'au moins une structure de type neuroépithéliale ; et
(iv_{NR}) cultiver les cellules obtenues à l'étape (iii_{NR}).

9. Procédé selon la revendication 8, dans lequel au moins une structure de type neuroépithélial est prélevée à l'étape (iii_{NR}).

10. Procédé selon l'une quelconque des revendications 8 à 9, dans lequel, à l'étape (iv_{NR}), le milieu de culture est enrichi en FGF2 pendant au moins 5 jours.
